(19) Europäisches Patentamt / European Patent Office / Office européen des brevets

(11) **EP 3 429 804 B1**

(12) **EUROPÄISCHE PATENTSCHRIFT**

(45) Veröffentlichungstag und Bekanntmachung des Hinweises auf die Patenterteilung:
**14.06.2023 Patentblatt 2023/24**

(21) Anmeldenummer: **17711616.7**

(22) Anmeldetag: **14.03.2017**

(51) Internationale Patentklassifikation (IPC):
**B25J 9/00** (2006.01)    **A61F 5/02** (2006.01)

(52) Gemeinsame Patentklassifikation (CPC):
**A61H 1/0281; A61F 5/026; B25J 9/0006;**
A61H 2201/165

(86) Internationale Anmeldenummer:
**PCT/EP2017/055998**

(87) Internationale Veröffentlichungsnummer:
**WO 2017/157941 (21.09.2017 Gazette 2017/38)**

(54) **EXOSKELETT FÜR EINEN MENSCHEN**

EXOSKELETON FOR A HUMAN BEING

EXOSQUELETTE POUR ÊTRE HUMAIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.03.2016 DE 102016003063**

(43) Veröffentlichungstag der Anmeldung:
**23.01.2019 Patentblatt 2019/04**

(73) Patentinhaber: **Exoiq GmbH**
**29581 Bohlsen (DE)**

(72) Erfinder:
• **WEIDNER, Robert**
**29581 Bohlsen (DE)**
• **WULFSBERG, Jens-Peter**
**22607 Hamburg (DE)**
• **OTTEN, Bernward**
**20535 Hamburg (DE)**
• **ARGUBI-WOLLESEN, Andreas**
**20146 Hamburg (DE)**

(74) Vertreter: **Maiwald GmbH**
**Elisenhof**
**Elisenstraße 3**
**80335 München (DE)**

(56) Entgegenhaltungen:
WO-A1-2014/195373    DE-A1-102011 076 843
JP-A- 2012 024 557    JP-A- 2012 239 818
JP-A- 2013 176 429

EP 3 429 804 B1

**Beschreibung**

Die Erfindung betrifft eine anziehbare und steuerbare Vorrichtung (Exoskelett) zur Vermeidung muskuloskelettaler Überlastungsschäden z.B. bei Tätigkeiten in oder über Kopfhöhe, beispielsweise bei Mitarbeitern des produzierenden Gewerbes und Handwerk, die besonders durch eine hohe Bewegungstreue (Kopplung rotatorischer und translatorischer Freiheitsgrade) aufgrund einer speziellen Gestaltung der kinematischen Struktur charakterisiert ist.

Hintergrund der Erfindung

Zahlreiche Anmeldungen beinhalten technische Systeme, um menschliche Tätigkeiten zu unterstützen, um z.B. die Qualität und die Ergonomie zu verbessern. Diese Systeme werden insbesondere zur Kraftverstärkung, und in unterschiedlichen Anwendungskontexten wie z.B. der Raumfahrt, verwendet. Damit lassen sich unterschiedliche Körperteile, z.B. der Arm oder der kompletten Körper, unterstützen.

Der Gegenstand der Erfindung ist vor allem durch eine deutlich intensivere und nähere, nicht nur temporäre Kopplung zwischen kinematischer Struktur und dem menschlichen Körperteil, insbesondere der Schulter, abgrenzbar. Diese Randbedingung führt zu einer deutlich schlankeren und kompakteren Gestaltung der Vorrichtung. Ersichtlich ist dies darin, dass in einer kompakten Vorrichtung neben den rotatorischen Freiheitsgraden auch die translatorischen Freiheitsgrade abgebildet werden. Hierdurch kann eine deutlich verbesserte Bewegungstreue realisiert werden.

Aus WO2014195373, DE102011076843, JP2012024557 und JP 20013-176429 A sind Exoskelette bzw. Rückenstützsysteme bekannt, die zum Teil auch lokal versteift werden können.

Zusammenfassung der Erfindung

Es ist eine Aufgabe der Erfindung eine Vorrichtung bereitzustellen, welche einen Menschen bei seinen Bewegungen, insbesondere bei über Kopf arbeiten, unterstützt.

Die Aufgabe wird durch die Merkmale des unabhängigen Patentanspruchs gelöst. Weitere Ausführungsformen der Erfindung ergeben sich aus den abhängigen Ansprüchen und der nachfolgenden Beschreibung.

Ein erster Aspekt der Erfindung betrifft ein Exoskelett für einen Menschen. Das Exoskelett weist auf:

- ein Schulteranlageelement;
- wenigstens eine Armstütze;
- ein Beckenstützelement; und
- ein Rückenteil, mit einem ersten Ende und einem zweiten Ende.

Das Rückenteil weist wenigstens ein erstes flächenflexibles Rückenelement mit einem ersten und einem zweiten Ende und wenigstens eine erste Versteifungseinrichtung mit einem ersten Versteifungselement auf, wobei das erste flächenflexible Rückenelement so ausgerichtet ist, dass dessen erstes Ende in Richtung des ersten Endes des Rückenteils gerichtet ist und dessen zweites Ende in Richtung des zweiten Endes des Rückenteils gerichtet ist. Das Rückenteil ist mit dem ersten Ende an dem Schulteranlageelement befestigt und ist mit dem zweiten Ende an dem Beckenstützelement befestigt. Die Armstütze ist direkt oder indirekt an dem Schulteranlageelement angelenkt. Die erste Versteifungseinrichtung ist ausgeführt, das erste flächenflexible Rückenelement gezielt in einer Biegerichtung zu versteifen.

Damit ein Nutzer bzw. der Mensch im Rahmen von seiner Tätigkeit, insbesondere bei über Kopf Arbeiten unterstützt werden kann, sieht die Erfindung ein Exoskelett vor. Dieses Exoskelett kann den Nutzer sowohl unterstützen als auch entlasten, in dem es die auftretende Kraft am Arm des Nutzers gezielt in den Rücken und das Becken des Nutzers leitet. Des Weiteren kann durch einen Aktuator der Nutzer bei seinen Bewegungen aktiv unterstützt werden.

Das Exoskelett gemäß der Erfindung kann im Wesentlichen aus drei Baugruppen aufgebaut sein. Als erste Baugruppe ist die Schultergelenksanordnung zur Nachbildung des Schultergelenks und der Unterstützung des Arms, insbesondere des Oberarms des Nutzers, zu nennen. Als zweite Baugruppe das Rückenteil, welches mit der Schultergelenksanordnung verbunden sein kann und zumindest partiell an dem Rücken des Nutzers anliegt. Als dritte Baugruppe ist das Beckenstützelement zu nennen. Es kann an dem der Schultergelenksanordnung gegenüberliegenden Ende des Rückenteils angeordnet sein. Das Rückenteil kann die Kraft aus der Schultergelenksanordnung über das Beckenstützelement in den Rücken und das Becken des Nutzers einleiten. So kann der Nutzer höhere Lasten bewältigen bzw. Arbeiten länger ausführen ohne seinen Körper zu schädigen bzw. zu überlasten.

Das Rückenteil kann einteilig aber auch mehrteilig ausgeführt sein.

Erfindungsgemäß weist das Rückenteil wenigstens ein flächenflexibles Rückenelement auf, das mittels einer Versteifungseinrichtung gezielt in einer Biegerichtung versteift werden kann. Die Versteifungseinrichtung kann ausgeführt sein, die Versteifung des flächenflexiblen Rückenelements in eine bevorzugte Biegerichtung auszuführen, d.h. die Versteifung erfolgt beispielsweise mit der Krümmungsrichtung des flächenflexiblen Rückenelements. Mit anderen Worten kann der Nutzer sich weiter Aufrichten, aber ein weiteres Krümmen des Rückens wird verhindert. Somit kann der Nutzer vor Haltungsschäden bewahrt werden und die auftretende Kraft in dem Schultergelenk des Nutzers kann zumindest teilweise in den Rücken und das Becken des Nutzers abgeführt werden.

In einer weiteren Ausführungsform kann ein flächenflexibles Rückenelement mehrere Versteifungseinrichtungen aufweisen, insbesondere wenn das Rückenteil nur ein flächenflexibles Rückenelement aufweist, kann es vorteilhaft sein auf diesem mehrere verschiedene Versteifungseinrichtungen anzubringen. Auch kann eine Versteifungseinrichtung an der Außenseite, also dem Nutzer abgewandt, und eine an der Innenseite angebracht werden.

Vorteilhafterweise ist an dem Rückenteil schulteranlagenelementseitig ein erster Kraftangriffspunkt vorgesehen, wobei an dem Rückenteil beckenstützelementseitig ein zweiter Kraftangriffspunkt vorgesehen ist, wobei das erste Versteifungselement ein Seil ist, das zwischen dem ersten Kraftangriffspunkt und dem zweiten Kraftangriffspunkt außerhalb der Biegelinie des ersten flächenflexiblen Rückenelements derart gespannt ist, so dass die Biegekraft gezielt in eine Richtung vergrößert wird.

In einer vorteilhaften Ausführungsform der Erfindung kann das Versteifungselement der Versteifungseinrichtungen ein gespanntes Seil sein. Dieses Seil kann zwischen zwei Kraftangriffspunkten über dem zu versteifenden flächenflexiblen Rückenelement gespannt werden. Durch die Eigenschaften des Seils, z.B. die Elastizität, und der Spannung des Seils, kann der Grad der Versteifung für das flächenflexible Rückenelement eingestellt werden. Das Seil verläuft typischerweise außen am Rückenteil entlang, also auf der dem Nutzer abgewandten Seite des Rückenteils, so dass der Nutzer nicht in Kontakt mit dem Seil steht. Das Seil kann auch beabstandet zu dem flächenflexiblen Rückenelement verlaufen, um einen größeren Hebel zu erreichen. Ein direktes Anliegen des Seils auf dem flächenflexiblen Rückenelement ist ebenfalls vorgesehen, um eine geringe Bauhöhe aufzuweisen. Die Kraftangriffspunkte können direkt in dem flächenflexiblen Rückenelement integriert sein, aber auch in anderen Komponenten des Rückenteils, wie z.B. dem Schulteranlageelement oder einem Verbindungselement.

Neben verschiedenen Seiltypen und Seilstärken kann das Versteifungselement auch aus einer Feder, Kunststoff, Metall, einem Pneumatikzylinder, einem Dämpfer, einem Gummiband, einem elektrischen Aktuator, einem piezoelektrischen Aktuator oder einem Stahlseil bestehen.

Gemäß der Erfindung weist das Rückenteil ferner ein zweites flächenflexibles Rückenelement mit einem ersten Ende und einem zweiten Ende, sowie ein Verbindungselement auf. Das zweite Ende des ersten flächenflexiblen Rückenelements ist mit dem ersten Ende des zweiten flächenflexiblen Rückenelements über das Verbindungselement verbunden.

Für eine gezieltere Versteifung des Rückenteils und einer besseren Anpassung an den Nutzer kann das Rückenteil auch mehrere flächenflexible Rückenelemente aufweisen. Insbesondere kann das Rückenteil zwei- oder dreiteilig ausgeführt sein. Das Rückenteil kann neben einem flächenflexiblen Rückenelement auch ein nicht flächenflexibles Rückenelement aufweisen.

Die Versteifung der jeweiligen flächenflexiblen Rückenelemente kann zum einen über das Material, die Materialstärke und die Geometrie des flächenflexiblen Rückenelements beeinflusst werden. Und zum anderen durch die gewählte Versteifungseinrichtung und deren Anbindung an das Rückenteil bzw. an das jeweilige flächenflexible Rückenelement.

Gemäß der Erfindung weist das zweite flächenflexible Rückenelement eine zweite Versteifungseinrichtung mit einem zweiten Versteifungselement auf, wobei die zweite Versteifungseinrichtung ausgeführt ist, das zweite flächenflexible Rückenelement gezielt in einer Biegerichtung zu versteifen. Die erste Versteifungseinrichtung und die zweite Versteifungseinrichtung weisen unterschiedlichen Steifigkeiten auf.

Für die individuelle Anpassung des Exoskeletts an den jeweiligen Nutzer kann bei einer Ausführungsform mit mehreren flächenflexiblen Rückenelementen und mehreren Versteifungseinrichtungen die Steifigkeit der einzelnen flächenflexiblen Rückenelemente an die jeweilige Situation unabhängig voneinander angepasst werden. Mit anderen Worten kann jede der verwendeten Versteifungseinrichtungen das jeweilige flächenflexible Rückenelement unterschiedlich versteifen und die Versteifungselemente der Versteifungseinrichtungen können unterschiedliche Spannungen aufweisen. Die Versteifungseinrichtungen können auch unabhängig voneinander geändert bzw. angepasst werden.

Gemäß einer Ausführungsform der Erfindung ist wenigstens eines von dem ersten flächenflexiblen Rückenelement und dem zweiten flächenflexiblen Rückenelement in Bezug auf das Verbindungselement längenverschiebbar angeordnet, so dass der Abstand zwischen dem ersten flächenflexiblen Rückenelement und dem zweiten flächenflexiblen Rückenelement veränderbar ist.

Für die Anpassung des Exoskeletts an den jeweiligen Nutzer und dessen Körpergröße, kann das Verbindungselement ausgeführt sein, dass sich der Abstand zwischen den, an diesem Verbindungselement angebrachten, flächenflexiblen Rückenelementen verändern lässt. Mit anderen Worten kann das Verbindungselement mehrere Anbindungspunkte für die flächenflexiblen Rückenelemente aufweisen. Hierbei kann ein flächenflexibles Rückenelement verschiebbar sein oder beide flächenflexible Rückenelemente können verschiebbar angeordnet sein. Ferner kann diese Funktionalität durch den Einsatz von Langlöchern erreicht werden. Vorteilhafter Weise ist das flächenflexible Rückenelement und das Verbindungselement trennbar miteinander verbunden, z.B. durch eine Schraube.

Gemäß einer Ausführungsform der Erfindung ist auf der dem Rückenteil abgewandten Oberfläche des flächenflexiblen Rückenelements von der Oberfläche wenigstens eine auskragende Haltestruktur angeordnet, an welcher das Versteifungselement der Versteifungseinrichtung anliegend ist, so dass das Versteifungselement beabstandet zu der Oberfläche des flächenflexiblen Rückenelements verläuft.

Zur Erzeugung eines größeren Hebels und somit einer größeren Versteifung des flächenflexiblen Rückenelements kann das Rückenteil eine auskragende Haltestruktur aufweisen. Es können auch mehrere Haltestrukturen pro flächenflexiblen Rückenelement und/oder für jedes flächenflexible Rückenelement eine Haltestruktur vorgesehen sein. Zum einen kann das Seil über die Haltestrukturen verlaufen und zum anderen mittels Bohrungen durch diese hindurch. Ferner kann die Haltestruktur dazu dienen das Seil umzulenken und somit eine mehr oder weniger parallele Anordnung des Seils zum flächenflexiblen Rückenelement zu erreichen. Die auskragende Haltestruktur kann direkt in das flächenflexible Rückenelement integriert sein, aber auch auf anderen Komponenten des Rückenteils angeordnet sein, wie z.B. auf dem Verbindungselement. Auch kann eine Versteifungseinrichtung über mehrere flächenflexible Rückenelemente gespannt sein.

Gemäß einer Ausführungsform der Erfindung weist wenigstens die erste Versteifungseinrichtung eine Seilspannvorrichtung auf, die ausgeführt ist, die Seilspannung zu verändern.

Ist eine höhere Steifigkeit des flächenflexiblen Rückenelements gewünscht bzw. erforderlich, kann die Seilspannung durch eine Seilspannvorrichtung erhöht werden. Bei einer Verringerung der Seilspannung wird auch die Steifigkeit des flächenflexiblen Rückenelements verringert. Für jede Versteifungseinrichtung kann eine gesonderte Seilspannvorrichtung vorgesehen sein. Die Seilspannvorrichtung kann manuell, also durch den Nutzer eingestellt werden, z.B. durch eine Verstellschraube, oder automatisch durch einen Aktuator.

Gemäß einer Ausführungsform der Erfindung weist wenigstens die erste Versteifungseinrichtung einen ersten Aktuator auf, wobei die Seilspannvorrichtung ausgeführt ist, mittels des ersten Aktuators die Seilspannung zu verändern.

Neben der manuellen Anpassung der Seilspannung durch die Seilspannvorrichtung kann auch ein automatisches bzw. ein unterstütztes Anpassen der Seilspannung mit Hilfe eines Aktuators erfolgen. Hierbei ist der Aktuator in die Seilspannvorrichtung integriert, so dass dieser bei Betätigung bzw. Ansteuerung die Spannung des Seils erhöhen oder verringern kann. In einer weiteren vorteilhaften Ausführungsform kann der Aktuator die Seilspannvorrichtung auch während des Betriebs des Exoskeletts situationsbedingt anpassen, d.h. die Spannung kann innerhalb einer Bewegung bzw. einer Aktion des Nutzers verändert werden, um sich so schnell an diverse Situationen anpassen zu können.

Gemäß einer Ausführungsform der Erfindung ist der erste Aktuator ein Pneumatikzylinder, ein pneumatischer Muskel oder ein Elektromotor.

Je nach Einsatzgebiet des Exoskeletts kann der Aktuator für das Verändern der Seilspannung unterschiedlich ausgeführt sein, d.h. der Aktuator kann pneumatisch, z.B. ein Pneumatikzylinder oder ein pneumatischer Muskel, elektrisch, piezoelektrisch oder hydraulisch betrieben werden.

Gemäß einer Ausführungsform der Erfindung weist das Exoskelett ferner

- eine Sensoranordnung zur Messung insbesondere eines Winkels oder einer Kraft; und
- eine Steuereinheit; auf.

Die Steuereinheit ist ausgeführt, den ersten Aktuator der Seilspannvorrichtung auf Basis von Sensordaten der Sensoranordnung zu steuern, so dass situationsbedingt die Seilspannung der Versteifungseinrichtung anpassbar ist.

Für eine gezielte Anpassung der Steifigkeit des flächenflexiblen Rückenelements, kann der Aktuator der Seilspannvorrichtung um eine Steuereinheit und einen Sensor erweitert werden. Der Sensor kann ausgeführt sein, eine Kraft auf das Exoskelett, insbesondere eine Kraft auf die Armstütze des Exoskeletts oder den Winkel des Arms des Nutzers in Bezug auf das Exoskelett zu messen. Das Steuergerät kann ausgeführt sein, den Aktuator auf Basis der Messwerte des Sensors anzusteuern und somit situationsbeding die Steifigkeit des flächenflexiblen Rückenelements derart anzupassen, dass der Nutzer optimal in seiner Bewegung bzw. Tätigkeit unterstützt wird. Hierdurch kann der Körper des Nutzers vor Überlastung geschützt werden.

Gemäß einer Ausführungsform der Erfindung sind das Rückenteil und das Beckenstützelement drehbar um eine erste Rotationsachse verbunden, welche orthogonal auf der Oberfläche des Rückenteils steht.

Zur Sicherstellung einer Lateralflexion des Nutzers, d.h. einem seitlichen Beugen des Nutzers, kann das Rückenteil mit dem Beckenstützelement über eine erste Rotationsachse verbunden sein. Die erste Rotationsachse kann hierbei senkrecht auf der Oberfläche des Rückenteils stehen. Dadurch kann die Bewegungsfreiheit des Nutzers in dem Exoskelett weiter gesteigert werden.

Gemäß einer Ausführungsform der Erfindung sind das Rückenteil und das Beckenstützelement drehbar um eine zweite Rotationsachse verbunden, welche quer zur Hauptrichtung des Rückenteils in der Fläche des Rückenteils verläuft.

Damit der Nutzer sich auch nach vorne beugen kann, kann eine zweite Rotationsachse in der Verbindung zwischen Rückenteil und Beckenstützelement vorgesehen sein. Die zweite Rotationsachse kann quer zur Hauptrichtung des Rückenteils liegen.

Gemäß einer Ausführungsform der Erfindung weist das Exoskelett ferner einen zweiten Aktuator auf, wobei der zweite Aktuator ein erstes Ende und ein zweites Ende aufweist. Das erste Ende des zweiten Aktuators ist mit dem Rückenteil verbunden, wobei das zweite Ende des zweiten Aktuators mit dem Beckenstützelementverbunden ist, wobei der zweite Aktuator ausgeführt ist, die Bewegung zwischen Rücken und Becken zu unterstützen.

Für eine optimale Unterstützung des Nutzers im unteren Wirbelsäulenbereich kann ein zweiter Aktuator vorgesehen sein, welcher die Bewegungen zwischen Beckenstützelement und dem Rückenteil unterstützt. Ferner können auch zwei Aktuatoren parallel neben der Wirbelsäule des Nutzers angeordnet sein, um den unteren Wirbelsäulenbereich zu unterstützen. Die Ansteuerung des zweiten Aktuators kann ebenfalls durch ein Steuergerät erfolgen, welches basierend auf Messdaten eines Sensors den Aktuator ansteuert. Der zweite Aktuator kann pneumatisch, elektrisch, piezoelektrisch oder hydraulisch betrieben werden.

Gemäß einer Ausführungsform der Erfindung besteht wenigstens das erste flächenflexiblen Rückenelement aus kohlefaserverstärktem Kunststoff.

Ferner können die anderen flächenflexiblen Rückenelemente auch aus kohlefaserverstärktem Kunststoff bestehen. Des Weiteren kann das flächenflexible Rückenelement aber auch aus anderen Materialien, wie beispielsweise Kunststoff, glasfaserverstärkter Kunststoff, Metall, Textilien (Geweben) oder aus einem Materialmix der vorgenannten bestehen.

Gemäß einer Ausführungsform der Erfindung weist das Exoskelett eine Schultergelenksanordnung auf, wobei eine Schultergelenksanordnung das Schulteranlageelement, ein erstes Schulterkopplungselement, ein zweites Schulterkopplungselement und die Armstütze aufweist. Das erste Schulterkopplungselement ist über eine erste Rotationsachse mit dem Schulteranlageelement verbunden. Das erste Schulterkopplungselement und das zweite Schulterkopplungselement sind über eine zweite Rotationsachse miteinander verbunden. Das zweite Schulterkopplungselement und die Armstütze sind über eine dritte Rotationsachse verbunden. Die erste Rotationsachse und die zweite Rotationsachse sind rechtwinklig zueinander und beabstandet voneinander angeordnet. Die zweite Rotationsachse und die dritte Rotationsachse schneiden sich.

Das Exoskelett kann auch zwei Schultergelenksanordnungen aufweisen, eine für die linke Schulter und die andere für die rechte Schulter des Nutzers. Zwischen dem zweiten Schulterkopplungselement und der Armstütze kann ferner ein Aktuator angebracht sein, welcher es ermöglicht die Rotation um die dritte Rotationsachse zu unterstützen.

Gemäß einer Ausführungsform der Erfindung weist die Schultergelenksanordnung ferner eine translatorische Achse entlang des Schulteranlageelements auf, wobei das erste Schulterkopplungselement entlang dieser translatorischen Achse verschiebbar ist.

Zur Anpassung des Exoskeletts an den Nutzer und insbesondere an die Schulterbreite des Nutzers kann eine translatorische Achse in dem Schulteranlageelement vorgesehen sein. Die translatorische Achse ermöglicht es das erste Schulterkopplungselement entlang des Schulteranlageelements zu verschieben. Die translatorische Achse kann in einer Ausführungsform auch arretiert werden, so dass die Schulerbreite des Nutzers eingestellt wird und anschließend das erste Schulterkopplungselement sich nicht mehr entlang der translatorische Achse verschieben lässt. Diese Arretierung ist lösbar ausgeführt, so dass eine Anpassung auf einen anderen Nutzer möglich ist. Als Alternative kann die translatorische Achse ohne Arretierung ausgeführt werden, somit kann ein weiterer Freiheitsgrad der Schultergelenksanordnung hinzugefügt werden.

Gemäß einer Ausführungsform der Erfindung ist die erste Rotationsachse eine Kippachse, die in einem Winkeln zwischen 0° bis 50° kippbar ist.

Gemäß einer Ausführungsform der Erfindung ist der Winkel zwischen der zweiten Rotationsachse und dritten Rotationsachse zwischen 0° und 90°, insbesondere beträgt der Winkel zwischen der zweiten Rotationsachse und der dritten Rotationsachse 85° $\pm$ 5°.

Gemäß einer Ausführungsform der Erfindung weist das Exoskelett ferner einen dritten Aktuator auf. Der dritte Aktuator weist ein erstes Ende und ein zweites Ende auf, wobei das erste Ende des dritten Aktuators mit der Armstütze verbunden ist und wobei das zweite Ende des dritten Aktuators mit dem zweiten Schulterkopplungselement verbunden ist. Der dritte Aktuator ist ausgeführt, die Schulterbewegung zu unterstützen.

Durch den Einsatz eines dritten Aktuators zwischen Armstütze und zweitem Schulterkopplungselement, kann der Nutzer bei seinen Bewegungen unterstützt werden. Insbesondere bei Arbeiten in oder über Kopfhöhe bzw. bei großen Schulterwinkel kann die Unterstützung erfolgen. Das Exoskelett kann ferner ausgeführt sein, die Versteifung des Rückenelements abhängig von der Unterstützungsleistung des dritten Aktuators zu steuern. Mit anderen Worten, je höher die Unterstützung der Schulter bzw. des Oberarms des Nutzers ist, desto mehr kann das flächenflexiblen Rückenelement versteift werden. In einer weiteren Ausführungsform kann die Steuerung des ersten Aktuators, des zweiten Aktuators und des dritten Aktuators durch die Steuereinheit durchgeführt werden. Die Steuereinheit kann für die Steuerung Messdaten des Sensors bzw. der Sensoren berücksichtigen.

Ferner kann die Vorrichtung gemäß der Erfindung an die jeweilige Körperabmessung angepasst werden, um die zu erwartenden Belastung zu stützen, d.h. z.B. zur Kraftsteigerung und -umleitung zumindest eines menschlichen Körperbereichs, bevorzugt einer Extremität. Diese Vorrichtung bzw. das Exoskelett ist durch den Nutzer anziehbar bzw. anlegbar und kann sowohl aus harten, als auch aus weichen oder weichen und harten Materialien gestaltet sein. Insbesondere das flächenflexible Rückenelement ist aus einem harten aber flexiblen Material, somit kann es sich an den Nutzer und dessen Statur anpassen. Weiche Komponenten können vor allem an Kontaktstellen zwischen Nutzer und Technik zum Einsatz kommen.

Dabei ist im Zusammenhang der Erfindung unter der Vorrichtung ein technisches System mit unterschiedlichen Systemelementen wie Mensch-Technik-Schnittstellen, wie z.B. eine Armstütze oder ein Beckenstützelement, (zur Kraftaufnahme und damit Übertragung der Kraft eines menschlichen Körperteils zum technischen Systems sowie zur Kraftübertragung vom technischen System zu einem menschlichen Körperteil), Verbindungselementen bzw. Vorrichtung zur Realisierung von rotatorischen und/oder translatorischen Bewegungen in menschlichen Körperteilen wie der Schulter-Arm-Einheit (auch bezeichnet als Mechanik), Sensoren und Aktuatoren zu verstehen. Die Vorrichtung zur Realisierung von rotatorischen und/oder translatorischen Bewegungen ist im Kontext dieser Offenbarung möglichst ähnlich zu dem menschlichen Gegenpart der unterstützt werden soll, z.B. die menschliche Schulter, und ist folglich anthropomorph zu gestalten. Die Vorrichtung zur Realisierung von menschlichen Bewegungen ist zum einen mit einer Mensch-Technik-Schnittstelle zur Kraftaufnahme, d.h. Übertragung der Kraft von einem menschlichen Körperteil, z.B. dem Oberarm, zum technischen System ausgestattet. Zum anderen ist die Vorrichtung mit einer Mensch-Technik-Schnittstelle ausgestattet, die die Kraft vom technischen System zurück in Strukturen des menschlichen Körpers überträgt, z.B. Rücken, Brust und/oder Becken. Diese Mensch-Technik-Schnittstelle kann beispielsweise eine dem menschlichen Torso nachgeahmte technische Vorrichtung, nachfolgend auch bezeichnet als Skelett des Torsos sein, welche nicht zwingend individualisiert ist, mit auf den Nutzer angepassten Krafteinleitungspunkten, z.B. mittels Verfahren des 3D-Drucks individuell hergestellt. Das Skelett des Torsos kann hierbei unterschiedlich gestaltet sein. Unterschiedliche Varianten hierfür werden nachfolgend beschrieben. Das Skelett des Torsos lässt sich z.B. mit einem Gurtsystem, wie bei einem Rucksack, relativ zum menschlichen Körper definiert anbringen. Darüber hinaus ist die Vorrichtung zur Realisierung menschlicher Bewegungen mit einer Antriebseinheit ausgestattet. Hierfür gibt es unterschiedliche passive und aktive Realisierungsmöglichkeiten; insofern aktiv, kann zudem eine Sensorik und Steuerungseinheit erforderlich sein. Die Antriebseinheit dient dazu, notwendige Kräfte und Momente in der Vorrichtung zu erzeugen, um menschliche Bewegungen zu unterstützen und nachzuahmen. Passiv könnte dies unter Berücksichtigung der spezifischen Eigenschaften z.B. durch eine mechanische Feder, Gasdruckfeder oder elastische Bänder realisiert werden. Zur aktiven Unterstützung lassen sich z.B. Elektromotoren, Pneumatik- und Hydraulikaktuatoren oder künstliche Muskeln einsetzen. Auch diese aktiven Antriebsmöglichkeiten weisen unterschiedliche Charakteristika auf. Die Anordnung der Antriebsmöglichkeiten kann zum einen direkt an der Rotations- oder Linearachse der Vorrichtung zur Realisierung menschlicher Bewegungen erfolgen. Zum anderen ist es ebenfalls möglich, die Antriebseinheiten dezentral zu platzieren und über z.B. eine Seilzugkinematik die Aktuierung zu realisieren.

Das Skelett des Torsos kann unterschiedlich gestaltet werden. Denkbar ist z.B. eine rein "maschinenähnliche Gestalt", beispielsweise aus einem Profilsystem um rein technische Funktionen realisieren zu können (z.B. einfaches und schnelles anpassen an geänderte Nutzerprofile). Eine weitere Möglichkeit wäre die Gestaltung in der Form, dass sich die Torsostruktur an die menschliche Wirbelsäule anpasst um einen optimalen Kraftfluss zu erreichen. Diese Struktur wird im Folgenden auch als S-Profil-System bezeichnet.

Auch die Gestaltung der Arm-Technik-Schnittstelle kann unterschiedlich erfolgen. Denkbar sind beispielsweise geschlossene und offene, harte und weiche Strukturen.

Die Vorrichtung ist zumindest mit zwei Körperteilen des Trägers direkt gekoppelt und wirkt direkt zusammen, beispielsweise mit dem Oberarm zur Kraftübertragung vom Menschen zum technischen System und zur Realisierung der Stützfunktion sowie mit dem Becken mittels eines Beckenstützelementes zur Kraftrückführung vom technischen System in menschliche Strukturen bzw. den menschlichen Körper. Die Kopplung erfolgt dabei bevorzugt mechanisch, indem z.B. die Vorrichtung durch den Nutzer angezogen werden kann und diese mit mindestens zwei bestimmten Teilen des menschlichen Körpers direkt und anliegend verbunden ist. Dabei kann die Vorrichtung mindestens eine Tätigkeit des Menschen, vornehmlich Hebe- und Handhabungsaufgaben, unmittelbar unterstützen, indem sie zum einen eine Kraftumleitung um besonders stark belastete Bereiche des menschlichen Körpers realisiert und zum anderen Stütz- und Bewegungsfunktionen durch eine gezielte Kraftaufbringung und - verstärkung realisiert. Somit dient das System zur Kraftaufnahme, Kraftumleitung, Kraftverstärkung und Krafteinleitung - und kann als Art Stützeinrichtung gesehen werden, beispielsweise für obere Extremitäten des Menschen.

Die Stützeinrichtung kann bei unterschiedlichen Bewegungsformen des Systemträgers unterstützen und soll Fehlbelastungen, beispielsweise menschlicher, oberer Extremitäten bei z.B. Tätigkeiten in oder über Kopfhöhe, vermeiden. Dennoch soll die Vorrichtung nicht die gesamte Arbeit des Trägers bzw. des Nutzers übernehmen oder ihn komplett entlasten. Das System kann zeitlich beschränkt oder permanent angeschaltet werden. Darüber hinaus sind mehrere Stufen der Intelligenz möglich. Variante eins sieht keine Möglichkeit vor das System an- oder auszuschalten, folglich ist das System (insofern es der Nutzer trägt) immer aktiv. Variante zwei ermöglicht es, dass System gezielt, d.h. durch den Nutzer, an und aus zu schalten (durch z.B. drücken einer Aktivierungstaste). Variante drei sieht eine Integration von Sensoren und einer Steuerungseinheit zur Verarbeitung der damit erzeugten Daten vor. Mit Hilfe dieser Informationen werden Belastungsdaten, z.B. über EMG- oder Kraftsensoren, genutzt um das System belastungsabhängig zu steuern. Beispielsweise ist das System bei geringen Belastungen inaktiv und wird aktiv hinzugeschaltet, sobald die Belastung einen kritischen Wert überschreitet.

Unterschiedliche Szenarien sollen mit dem Gegenstand der Erfindung abgedeckt werden, die nachfolgend in Use Cases beschrieben werden.

Use Case 1 sieht den Einsatz des Systems vor, um eine Überlastung, beispielsweise des Schulter-Arm-Bereichs, zu vermeiden. In diesem Fall dient die Vorrichtung zur Kraftumleitung. Je nach Ausführungsform ist das System permanent oder zeitweise im Einsatz. Insofern das System eine Intelligenz besitzt, soll das System im Fall einer Überlastung mit negativen Effekten auf die Gesundheit (vorzeitiger Gelenkverschleiß, etc.) die Überlast für den Träger um den kritischen Betrag reduzieren, um wieder auf ein physiologisch verträgliches Maß der Belastung zu kommen. Ziel des Systems ist daher eine gesteuerte Reduktion von Überlast für den Träger, und nicht eine komplette Entlastung. Die physiologisch vertretbare Belastung des Trägers ist aufgrund der positiven Effekte auf des Trägers Gesundheit (Gesundheitserhalt) explizit erwünscht. Prinzipiell, d.h. insofern die Vorrichtung optimal auf den Nutzer angepasst ist, schränkt die Vorrichtung menschliche Bewegungen nicht ein: Mensch und Technik sind räumlich und zeitlich synchronisiert und führen die Bewegung gemeinsam aus. Im Fall einer Überlastung würde die Vorrichtung, die parallel zur belasteten Region des Menschen angeordnet ist, die Kraft gezielt umleiten. Die Steuerung der Vorrichtung bei menschlichen Bewegungen und geänderten Beanspruchungsprofil kann hierbei wie oben beschrieben unterschiedlich erfolgen. Der Aufbau der Vorrichtung ist hierbei angepasst an den Systemträger und die durchzuführende Aufgabe.

Use Case 2 sieht den Einsatz des Systems rein zur Stabilisierung und Kraftsteigerung für ergonomisch ungünstigen Tätigkeiten wie Tätigkeiten in oder über Kopfhöhe vor, um dem Nutzer die Ausführung der Tätigkeit überhaupt zu ermöglichen. In diesem Fall dient die Vorrichtung primär zur gezielten Krafterzeugung zur Verstärkung einer Bewegung oder zur gezielten Stabilisierung. Sekundär soll die Kraft zudem umgeleitet werden (siehe Use Case 1). Je nach Ausführungsform ist auch in Use Case 2 das System permanent oder zeitweise aktiv. Insofern das System eine Intelligenz besitzt, soll das System nur bei kritischen Aufgaben unterstützen und die Unterstützung auf ein Minimum reduzieren, insofern eine Unterstützung "nicht unbedingt notwendig ist". Ziel des Systems ist daher eine gesteuerte Unterstützung der Bewegung des Systemträgers. Im Fall einer menschlichen Bewegung, z.B. Rotation oder Absenken oder Anheben der Schulter oder Heben des Armes, würde die Vorrichtung, die parallel zur belasteten Region des Menschen angeordnet ist, die für die Bewegung notwendige Kraft (bei intelligenten Systemen nur insofern erforderlich) aufbringen und insofern die Bewegung vernachlässigbar klein ist, die notwendige Kraft zum Stabilisieren aufbringen. Die Steuerung der Vorrichtung bei menschlichen Bewegungen und geänderten Beanspruchungsprofil kann hierbei wie oben beschrieben unterschiedlich erfolgen. Der Aufbau der Vorrichtung ist hierbei angepasst an den Systemträger und die durchzuführende Aufgabe.

Bei dem Gegenstand der Erfindung liegt ein besonderer Kern in der kinematischen Struktur der Vorrichtung, die menschliche rotatorische und lineare Bewegungen ohne Einschränkung durch eine körpernahe Vorrichtung ermöglicht, z.B. Rotation, Absenken und Anheben der Schulter.

Allgemein wird durch das Gesamtkonzept, vor allem für die Vorrichtung zur Realisierung rotatorischer und translatorischer menschlicher Bewegungen, eine Komplexitätsreduktion erreicht, da in erster Linie Bewegungen in vertikaler Richtung betrachtet werden und die Anforderungen an die zu verwendende Aktorik und Sensorik (zweites vornehmlich für aktive Systeme) folglich für Bewegungen in horizontaler Richtung minimiert werden.

Eine weitere Besonderheit liegt in der Steuerung. Das System greift je nach gewählter Steuerungsvariante nur aktiv ein, insofern je nach Use Case eine Überlastung vermieden werden soll oder eine Unterstützung zur Ausführung einer Aufgabe erforderlich ist. Ein Beispiel für die Vermeidung der Überlast ist die manuelle Lasthandhabung. Hierbei würde das System erst dann in die Lastenhandhabungen eingreifen, wenn der Träger entweder das System aktiv startet, da er um die Überlast weiß, oder aber wenn das System so ausgestaltet ist, dass es die Überlast automatisch detektiert. Die Unterstützung von Tätigkeiten in oder über Kopfhöhe ist ein Beispiel für die Unterstützung der Ausführung einer Aufgabe. Das Vorgehen ist äquivalent zu dem des ersten Beispiels.

Die Vorrichtung kann je nach Gestaltung aus unterschiedlichen Elementen, in jeweils unterschiedlicher Anzahl, bestehen. Die Konfiguration, d.h. der Zusammenbau der Elemente zu einem Gesamtsystem bzw. einer Vorrichtung, und die Auswahl der Elemente erfolgt in Abhängigkeit zu den Anforderungen, die durch den Systemnutzer und die Aufgabe maßgeblich bestimmt werden. Die Kopplung zwischen System und Nutzer kann unterschiedlich erfolgen, z.B. als Rucksack, Tragetasche oder Kleidungsstück. Zum einen kann das System Körperteile vor Überlastung schützen, indem sie die auf die menschlichen Körperstellen lastenden Kräfte umleitet. Zum anderen kann das System menschliche Bewegungen unterstützen bzw. ermöglichen und Positionen stabilisieren. Eine typische Aufgabe hierbei sind Tätigkeiten in oder über Kopfhöhe in der industriellen Produktion oder dem Handwerk. Das System besteht aus zumindest einer Vorrichtung zur Realisierung biomechanischer und translatorischer menschlicher Bewegungen und zumindest zwei Mensch-Technik-Schnittstellen - wobei eine zur Kraftaufnahme, d.h. Übertragung der Kraft von einer menschlichen Struktur zum technischen System, und eine zur Kraftableitung, d.h. Übertragung der Kraft von dem technischen System zur menschlichen Struktur - sowie einer aktuierenden Einheit bzw. einem Aktuator. Eine Ergänzung um z.B. eine Sensor- und Steuerungseinheit ist möglich und bei gewissen Gestaltungsvarianten, z.B. bei aktiven Antriebseinheiten, erforderlich.

Die Vorrichtung kann vor allem zur Unterstützung von Menschen eingesetzt werden, die ergonomisch ungünstige oder aber repetitive bzw. über einen längeren Zeitraum zu erfüllende Aufgaben ausführen müssen. Darüber hinaus ist der Einsatz zur Stabilisierung von zumindest Teilen elastischer oder nachgiebiger technischer Elemente oder technischer Gelenke oder die Unterstützung anderer Lebewesen mittelfristig vorgesehen.

Durch die Erfindung wird somit eine Vorrichtung geschaffen, die zumindest einen Teil eines Körpers einer Person oder eines anderen Lebewesens oder eines technischen Systems (wie ein Industrieroboter) durch eine Kraftumleitung und -verstärkung entlastet bzw. schont, und die Ausführung gewisser Tätigkeiten überhaupt erst ermöglicht.

Für die Auslegung, Konstruktion und Konfiguration der Vorrichtung sind die kinematischen Gegebenheiten, d.h. insbesondere die Anatomie des relevanten Köperteils des Nutzers und die Aufgabe zu berücksichtigen. Hierbei ist vor allem an die zwei Arten der Mensch-Technik-Schnittstelle, die Vorrichtung zur Realisierung rotatorischer und translatorischer menschlicher Bewegungen sowie die Aktuatorik und eventuelle Sensorik inkl. Steuerung zu denken, die maßgeblich die Leistungsfähigkeit bestimmen. Diese Elemente können starr oder flexibel miteinander verbunden werden, so dass die Vorrichtung unterschiedlich viele Freiheitsgrade besitzen kann. Unterschiedlichste geometrische Gestaltungsmöglichkeiten sind möglich.

Der Gegenstand der Erfindung führt nur zu geringen Zwangskräften an den Mensch-Maschine-Schnittstellen, da die kinematische Struktur auf die Biomechanik des Nutzers der Stützvorrichtung angepasst ist (hierdurch hohe Akzeptanz durch Hoheit beim Anwender). Aufgrund seiner Struktur ermöglicht das System eine gezielte Krafteinleitung in das technische System, eine gezielte Kraftumleitung um kritische menschliche Strukturen und das gezielte Einleiten einer Kraft vom technischen System in das technische System. Damit bewegt sich das Kernstück dieser Erfindung parallel zu der menschlichen Biomechanik und ist bevorzugt anthropomorph gestaltet. Eine andere Gestaltung ist jedoch auch möglich. Besonders hervorzuheben ist eine mögliche Ausprägungsform des Skeletts des technischen Torsos (bezeichnet als S-Profil-System), welche die Anpassbarkeit des Systems an die physiologische Doppel-S Form der Wirbelsäule gewährleistet, demnach gestalterisch angepasst an die Anordnung von Brustkyphose und Lendenlordose.

Zusammengefasst ist die Vorrichtung mit einer oder mehreren Schnittstellen zwischen Vorrichtung und Nutzer (bevorzugt zwei), mit einer oder mehreren Vorrichtung/en zur Realisierung rotatorischer und translatorischer menschlicher Bewegungen (bspw. der Schulter) und einer passiven und/oder aktiven Antriebseinheit, im aktiven Fall mit einer mit einer Sensoreinrichtung und Steuerungseinheit, ausgestattet. Die Vorrichtung ermöglicht zum einen eine ergonomischere günstigere Ausführung manueller Tätigkeiten z.B. in oder über Kopfhöhe, und zum anderen kann hierdurch die Ausführung von körperlich anstrengenden oder kritischen Aufgaben ermöglicht werden. Insbesondere kann durch das System die Gewichtskraft der durch den Nutzer gehaltenen Objekte durch Unterstützung an den oberen Extremitäten ausgeglichen werden. Durch die gezielte Anordnung aktiv angetriebener und passiver Freiheitsgrade zumindest eines Teils der Vorrichtung zur Realisierung rotatorischer und translatorischer menschlicher Bewegungen ist es in einer vorstellbaren Ausprägungsform möglich, vor allem vertikal wirkende Kräfte von der oberen Extremitäten in den Torso umzuleiten. Wohingegen in horizontaler Richtung der Nutzer nur gering eingeschränkt wird. Somit ist es möglich die schädliche Gewichtslaste konstruktiv von der aus gesundheitlichen Gründen nützlichen und für die Handhabung wichtigen Bewegung in der horizontalen Ebene zu trennen.

Durch derartige Systeme können Mitarbeiter in unterschiedlichen Anwendungskontexten nachhaltig in ihrem Arbeitsumfeld integriert werden, da hierdurch zum einen der vorzeitige Verschleiß verringert werden kann und zum anderen diese Systeme die Verminderung der körperlichen Leistungsfähigkeit verringern können. Das System kann sowohl im beruflichen Sektor, z.B. bei Hebe- und Tragevorgänge, als auch im privaten Bereich, z.B. bei der Installation von Lampen und beim Malern eingesetzt werden.

Bei dem Gegenstand der Erfindung handelt es sich um ein tragbares, insbesondere um ein anziehbares System zur Vermeidung von Überlastungsschäden und Unterstützung ergonomisch ungünstiger Tätigkeiten. Für die konkrete Systemgestaltung gibt es unterschiedliche Ausprägungsvarianten. Die Beweglichkeit des Nutzers, also die menschliche Bewegung, wird durch das System i.d.R. nicht beeinflusst bzw. eingeschränkt. Der Aufbau des Systems kann aus unterschiedlichen Materialien, die weich oder hart sein können, erfolgen, wie beispielsweise Aluminium, Textilien, GFK, CFK oder Kunststoff.

Zusammenfassen lässt sich, dass sich der Gegenstand der Erfindung vor allem durch folgende Aspekte auszeichnet:

- Die gezielte Kopplung von rotatorischen und translatorischen Freiheitsgraden in einer kinematischen Struktur führt zu einer sehr hohen Bewegungstreue des Unterstützungssystems.
- Ein weiterer großer Vorteil der Vorrichtung kann in der deutlich einfacheren, kompakteren und kostengünstigeren Bauweise gesehen werden.
- Für die Auslegung der Kinematik wurden speziell Messungen (insbesondere aus biomechanischen Analysen) berücksichtigt, um ein anthropomorphes Design des körpernahen/körpergetragenen Systems zu realisieren. Dies führt dazu, dass die Bewegungstreue trotz der Reduzierung der Freiheitsgrade sehr gut nachgebildet werden kann.

- Die modulare Struktur und die speziell vorgesehenen Anpassungsmöglichkeiten ermöglichen eine optimale Nutzung der erfinderischen Vorrichtung.

Durch vernachlässigbare Zwangskräfte (hiermit Hoheit über Anwendung bei Nutzer), bedingt durch die kinematische Struktur bzw. Aktorik, die vornehmlich die Gewichtskraft kompensiert, sowie der sehr guten Anpassungsmöglichkeit und der sehr körpernahen und kompakten Systemgestalt ergibt sich eine hohe Akzeptanz.

Kurze Figurenbeschreibung

Ausführungsbeispiele der Erfindung sind in den Zeichnungen dargestellt und werden im nachfolgenden näher beschrieben.

Fig. 1                    zeigt eine schematische Darstellung eines Exoskeletts in einer seitlichen Ansicht.

Fig. 2                    zeigt eine schematische Darstellung eines Exoskeletts mit einer beabstandeten Versteifungseinrichtung in einer seitlichen Ansicht.

Fig. 3                    zeigt eine schematische Darstellung eines Exoskeletts mit einer beabstandeten Versteifungseinrichtung in einer isometrischen Ansicht gemäß der Erfindung.

Fig. 4 aus Figur 3    zeigt eine schematische Darstellung des Exoskeletts in einer seitlichen Ansicht.

Fig. 5                    zeigt eine Schultergelenksanordnung gemäß einer Ausführungsform der Erfindung.

Fig. 6                    zeigt die erste Rotationsachse, welche eine Kippachse ist, gemäß einer Ausführungsform der Erfindung.

Fig. 7                    zeigt eine Schultergelenksanordnung in einer isometrischen Ansicht gemäß einer Ausführungsform der Erfindung.

Fig. 8                    zeigt ein erstes Beispiel für eine erfindungsgemäße Stützvorrichtung, deren Zusammenwirken mit einer Person beim Ausführen einer Tätigkeit in und über Kopfhöhe in Körperseitenansicht

Fig. 9                    zeigt ein erstes Beispiel für eine erfindungsgemäße Stützvorrichtung, deren Zusammenwirken mit einer Person in Ruhestellung in Körperrückansicht,

Fig. 10                   zeigt ein zweites Beispiel für eine erfindungsgemäße Stützvorrichtung, deren Zusammenwirken mit einer Person in Ruhestellung in Körperrückansicht,

Fig. 11                   zeigt ein drittes Beispiel für eine erfindungsgemäße Vorrichtung, deren Zusammenwirken mit einer Person in Ruhestellung in Körperrückansicht

Fig. 12                   zeigt eine Explosionszeichnung der erfindungsmäßen Vorrichtung gemäß einer Ausführungsform der Erfindung.

Beschreibung von Ausführungsformen der Erfindung

Ein bevorzugtes Einsatzgebiet der erfindungsgemäßen Stützvorrichtung ist die Unterstützung körperlich arbeitender Menschen im privaten und beruflichen Umfeld bei ergonomisch kritischen Aufgaben wie Tätigkeiten in oder über Kopfhöhe, z.B. Installations- und Montageaufgaben bei Handwerkern, in der Automobil- und Luftfahrtindustrie. Dies sind durchweg Tätigkeiten, die sowohl heutzutage, als auch in Zukunft, bevorzugt durch den Menschen ausgeführt werden. Der Bedarf hierfür wird z.B. aufgrund des demografischen Wandels, der gestiegenen Anforderungen im Produktionsumfeld durch komplexere und individualisiertere Produkte immer größer. Die erfindungsgemäße Stützvorrichtung kann dazu führen, dass diese Tätigkeiten aufgrund einer gezielten Entlastung und damit einhergehenden Reduktion von Überlastungsschäden über einen längeren Zeitraum als bisher durchgeführt werden können und zudem gewisse Tätigkeiten, die besondere Fertigkeiten verlangen, erst ermöglicht (z.B. Handhabung schwerer Lasten). Des Weiteren kann sie Tätigkeiten für Personengruppen erleichtern, die bislang für diese Belastungsgrößen nicht optimal ausgerüstet sind. Diese Anwendungsbeispiele zeigen, dass die Thematik eine große gesellschaftliche Relevanz besitzt (nachhaltiger Einsatz der Ressource Mensch im privaten und beruflichen Umfeld). Die Lücke einer notwendigen Systemtechnik zur zielgerichteten Unterstützung kann durch die Stützvorrichtung minimiert werden ohne den Menschen durch ein technisches System zu ersetzen. Als Folge dessen kann die gesamte Volkswirtschaft gestärkt werden, da z.B. die Krankheitstage je Arbeitnehmer aufgrund der Vermeidung von Fehlbelastungen verringert werden können.

Der Gegenstand der Erfindung unterstützt den Anwender durch eine anziehbare Stützvorrichtung, die parallel zu mindestens einem menschlichen Körperteil angeordnete, auf unterschiedlicher Weise steuerbare parallel angeordnete Elemente aufweist, die in Summe eine Stützvorrichtung darstellen. Hierzu können auch Funktionen zur Regelung und Steuerung der Vorrichtungselemente und dafür notwendige Sensorik gehören.

Im nachfolgenden werden Ausführungsformen anhand eines Anwendungsfalls, Tätigkeiten in und über Kopfhöhe, der erfindungsgemäßen Vorrichtung dargestellt.

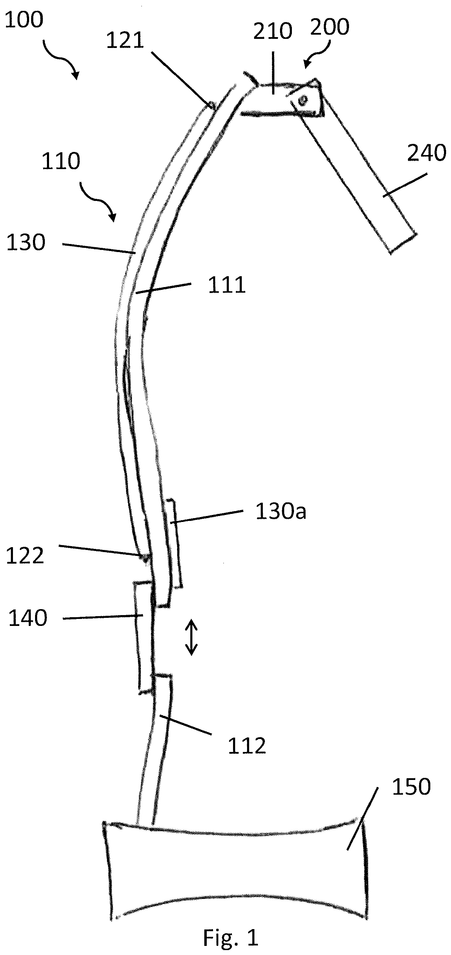

Fig. 1 zeigt ein Exoskelett 100 in einer seitlichen Ansicht. Das Exoskelett 100 in Fig. 1 weist mehrere Baugruppen auf, diese sind zum einen das Rückenteil 110, die Schultergelenksanordnung 200 und das Beckenstützelement 150. Das Rückenteil 110 verbindet hierbei das Beckenstützelemente 150 mit der Schultergelenksanordnung 200. Die Schultergelenksanordnung 200 umfasst wenigstens eine Armstütze 240 und ein Schulteranlageelement 210. Das Rückenteil 110 weist wenigstens ein erstes flächenflexibles Rückenelement 111 auf. Dieses ist senkrecht zur Hauptausdehnungsrichtung flexibel gestaltet, so dass ein Nutzer das erste flächenflexible Rückenelement 111 verbiegen kann. Das erste flächenflexible Rückenelement 111 ist direkt oder indirekt mit dem Beckenstützelement 150 verbunden und ist mit der Schultergelenksanordnung 200 verbunden. Bei einer indirekten Verbindung zwischen dem ersten flächenflexiblen Rückenelement 111 und dem Beckenstützelement 150, kann ein zweites flächenflexibles Rückenelement 112 und/oder ein Verbindungselement 140 die Verbindung zwischen dem ersten flächenflexiblen Rückenelement 111 und dem Beckenstützelement 150 herstellen, wobei der Abstand zwischen dem ersten flächenflexiblen Rückenelement 111 und dem zweiten flächenflexiblen Rückenelement 112 über das Verbindungselement 140 einstellbar ist. Somit kann das Exoskelett 100 auf Nutzer mit verschiedener Statur angepasst werden. Auf dem ersten flächenflexiblen Rückenelement 111 kann eine erste Versteifungseinrichtung 130 angebracht sein, welche es ermöglicht das ersten flächenflexiblen Rückenelement 111 gezielt in einer Biegerichtung zu versteifen. Die erste Versteifungseinrichtung 130 kann Außen in Bezug auf den Rücken des Nutzers auf dem ersten flächenflexiblen Rückenelement 111 angebracht sein. Das erste Versteifungselement 130 ist durch zwei Kraftangriffspunkte 121, 122 mit dem ersten flächenflexiblen Rückenelement 111 verbunden. Der erste Kraftangriffspunkt 121 ist hierbei schultergelenksanordnungsseitig angebracht und der zweite Kraftangriffspunkt 122 ist beckenstützelementseitig angebracht.

In Fig. 1 ist ferner eine innere Versteifungseinrichtung 130a auf der Innenseite des flächenflexiblen Rückenelements 111 angebracht, so dass auch die zweite Biegerichtung gezielt versteift werden kann. Des Weiteren können sich die Bereiche der ersten Versteifungseichrichtung 130 und der inneren Versteifungseinrichtung 130a überlappen, so dass das erste flächenflexible Rückenelement sowohl in die erste Biegerichtung als auch in die zweite Biegerichtung versteift ist.

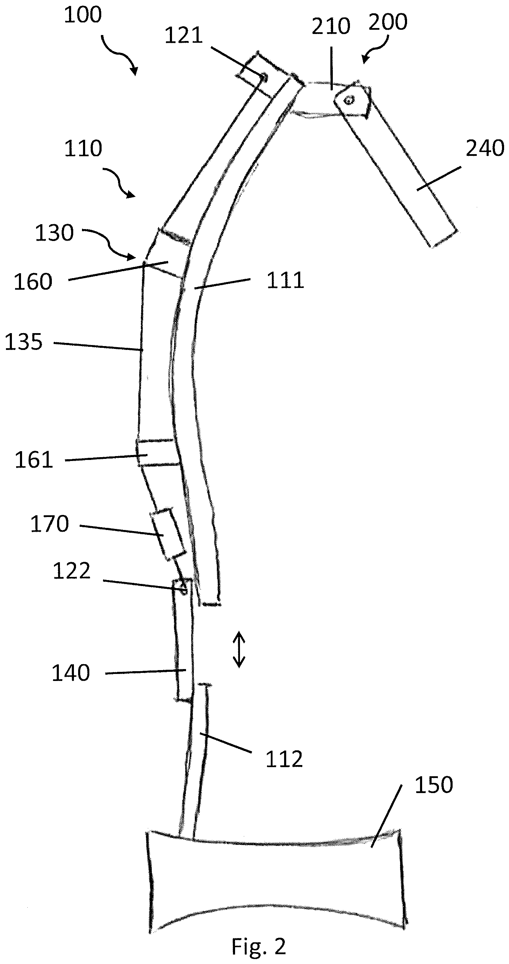

Fig. 2 zeigt im Wesentlichen das Exoskelett 200 aus Fig. 1. Auch in Fig. 2 verfügt das Exoskelett über eine Schultergelenksanordnung 200 mit einer Armstütze 240 und einem Schulteranlageelement 210. Ferner weist das Exoskelett 100 ein Rückenteil 110 und ein Beckenstützelement 150 auf, wobei das Rückenteil 110 das Beckenstützelement 150 mit der Schultergelenksanordnung 200 verbindet. Als Unterschied zu Fig. 1 weißt Fig. 2 eine andere Ausprägung der Versteifungseinrichtung 130 auf. Die Versteifungseinrichtung 130 weist ferner ein Versteifungselement 135 auf, wobei dieses in Fig. 2 zwischen einem ersten Kraftangriffspunkt 121 und einem zweiten Kraftangriffspunkt 122 angeordnet ist. Der erste Kraftangriffspunkt 121 befindet sich auf dem Schulteranlageelement 210. Der zweite Kraftangriffspunkt 122 befindet sich auf dem Verbindungselement 140. Des Weiteren sind auf dem ersten flächenflexiblen Rückenelement 111 auskragende Haltestrukturen 160, 161 angebracht, über die das Versteifungselement 135 verläuft. Das Versteifungselement 135 kann z.B. ein gespanntes Seil oder eine Feder sein. Durch die Haltestrukturen 160, 166 kann der Abstand zwischen dem Versteifungselement 135 und dem zu versteifenden ersten flächenflexiblen Rückenelement 111 eingestellt werden, so dass eine gezieltere Versteifung des ersten flächenflexiblen Rückenelements sichergestellt werden kann. Ferner weist die Versteifungseinrichtung 130 eine Seilspannvorrichtung 170 auf. Diese Seilspannvorrichtung 170 ermöglicht es die Seilspannung bzw. die Federspannung anzupassen. Die Anpassung kann hierbei manuell oder automatisch erfolgen. Die Seilspannung kann vorteilhafterweise an die Bedürfnisse des Nutzers, z.B. an dessen Körpergröße, Muskelstärke, und an die zu verrichtende Tätigkeit angepasst werden. Für eine automatische Anpassung der Seilspannung kann ein Aktuator in der Seilspannvorrichtung 170 vorgesehen sein. Dieser kann beispielsweise ein Pneumatikzylinder, ein Hydraulikzylinder, ein pneumatischer Muskel, ein Piezoelement oder ein Elektromotor sein. Des Weiteren ist es möglich, eine Steuereinheit und eine Sensoranordnung zur Steuerung des Aktuators einzusetzen. Somit kann der Aktuator an die jeweilige Situation angepasst werden. D.h. abhängig von den auftretenden Kräften bzw. Winkeln an dem Exoskelett 100 kann die Versteifung der Versteifungseinrichtung 130 angepasst werden. Dadurch kann die Steifigkeit des Rückteils 110 angepasst werden und der Nutzer kann individuell unterstützt werden.

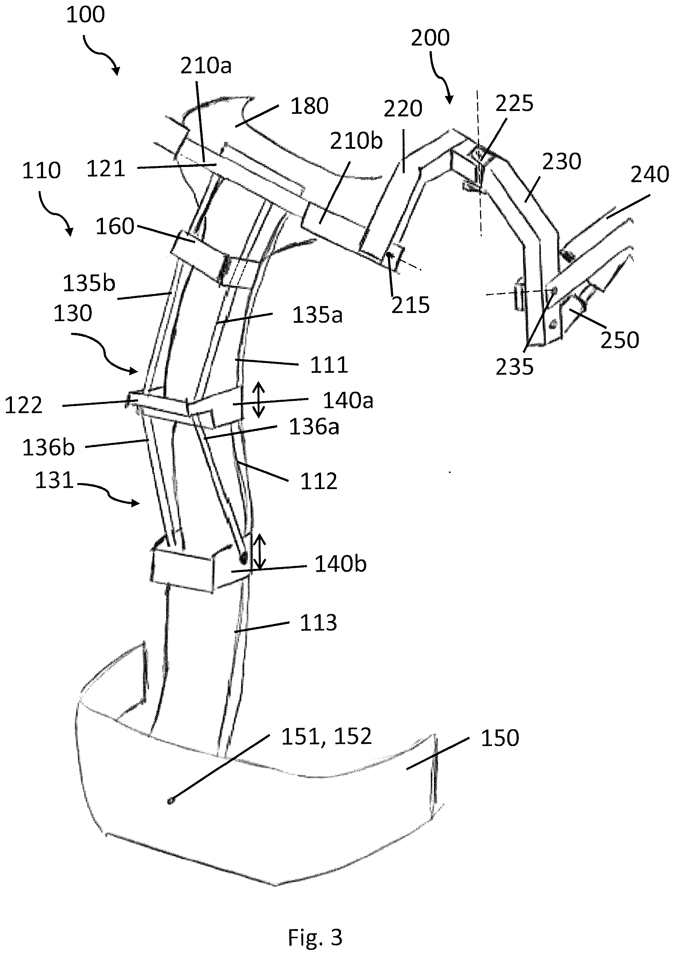

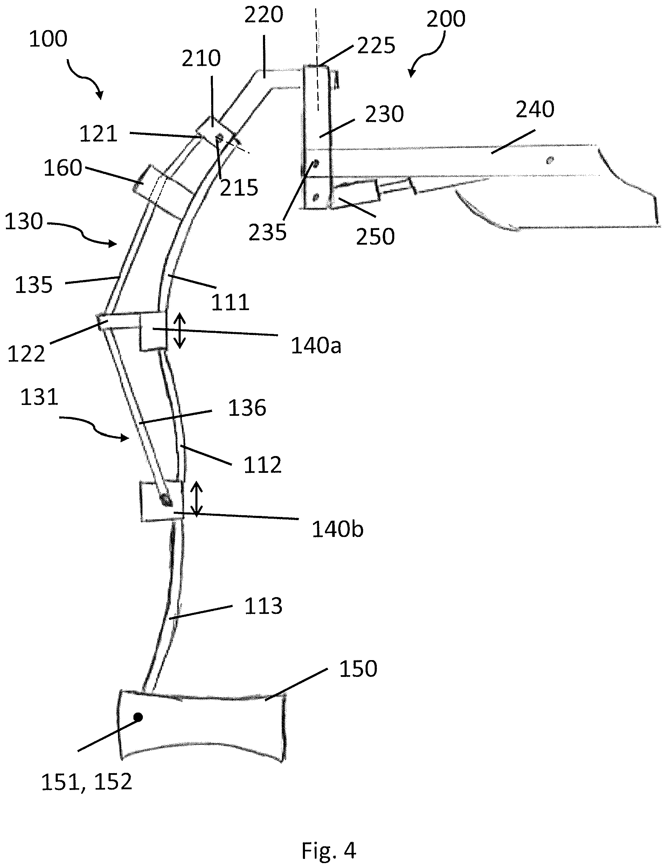

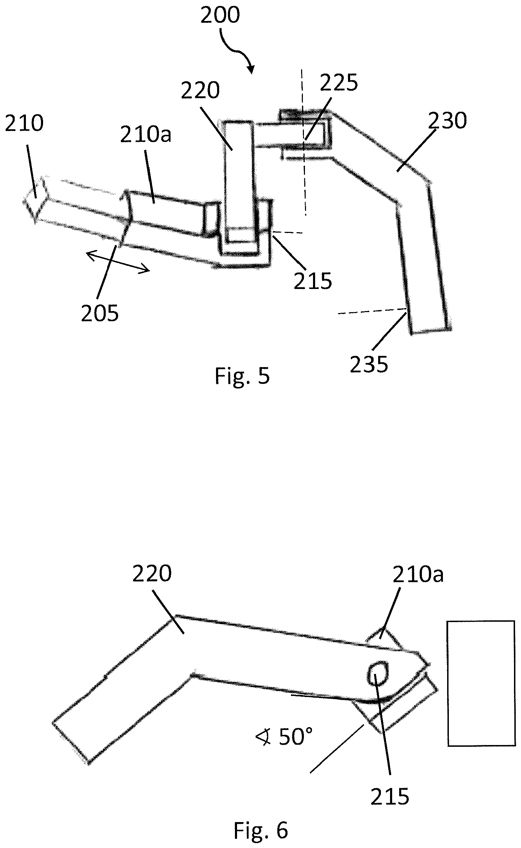

Fig. 3 und Fig. 4 zeigen ein Exoskelett 100 gemäß der Erfindung in einer isometrischen bzw. einer seitlichen Ansicht. Im Gegensatz zu den Figuren 1 und 2 ist das Rückenteil 110 in Fig. 3 dreiteilig ausgeführt, d.h. es sind drei flächenflexible Rückenelemente 111, 112, 113 vorhanden. Diese drei flächenflexiblen Rückenelemente 111, 112, 113 sind über zwei Verbindungselemente 140a, 140b verbunden. Die Verbindungselemente 140a, 140b ermöglichen es den Abstand zwischen den jeweils verbundenen flächenflexiblen Rückenelementen einzustellen, so dass eine Höhenanpassung an den jeweiligen Nutzer ermöglicht wird. Des Weiteren weist Fig. 3 ein Schulterauflage 180 auf, diese dient dazu das Exoskelett bequem für den Nutzer auf dessen Schultern zu platzieren. Die Verbindung zwischen dem dritten flächenflexiblen Rückenelement 113 und dem Beckenstützelement 150 erfolgt über eine Anordnung, welche eine erste Rotationsachse 151 und eine zweite Rotationsachse 152 aufweist. Die erste Rotationsachse 151 ermöglicht es dem Nutzer seinen Rücken seitlich zu beugen, also eine Lateralflexion des Rückens. Die zweite Rotationsachse 152 ermöglicht es dem Nutzer sich vorzubeugen, also ein Beugen des Beckenstützelements 150 gegenüber dem dritten flächenflexiblen Rückenelements 113. In Fig. 3 ist die erste Versteifungseinrichtung 130 mit zwei parallelen Versteifungselementen 135a, 135b ausgeführt, welche über dem ersten flächenflexiblen Rückenelement 111 gespannt sind. In diesem Fall handelt es sich bei den Versteifungselementen 135a, 135b um gespannte Seile. Ein erster Kraftangriffspunkt 121 befindet sich, wie bei Fig. 2, an dem Schulteranlageelement 210. Der zweite Kraftangriffspunkt 122 der ersten Versteifungseinrichtung 130 befindet sich an dem Versteifungselement 140a. Die Versteifungselemente 135a, 135b werden durch die Haltestruktur 160 hindurch geführt, um eine Verbindung zwischen dem ersten Kraftangriffspunkt 121 und dem zweiten Kraftangriffspunkt 122 herzustellen. Des Weiteren wird durch die Haltestruktur 160 ein Abstand zwischen den Versteifungselementen 135a, 135b dem ersten flächenflexiblen Rückenelement 111 hergestellt. Durch diese Anordnung kann die erste Versteifungseinrichtung 130 das erste flächenflexible Rückenelement 111 gezielt versteifen. Eine zweite Versteifungseinrichtung 131 ist über dem zweiten flächenflexiblen Rückenelement 112 angeordnet. Auch die zweite Versteifungseinrichtung 131 weist zwei parallele Versteifungselemente 136a 136b auf. Die Versteifungselemente 136a, 136b sind zwischen den beiden Verbindungselementen 140a, 140b gespannt und sind ausgeführt das zweite flächenflexible Rückenelement 112 gezielt zu versteifen. Die Seilspannung der einzelnen Versteifungselemente 135a, 135b, 136a, 136b kann unterschiedlich ausgeführt werden, aber auch gleich. Ferner weist Fig. 3 eine Schultergelenksanordnung 200 auf, welche in den Fig. 5 - 7 näher beschrieben wird.

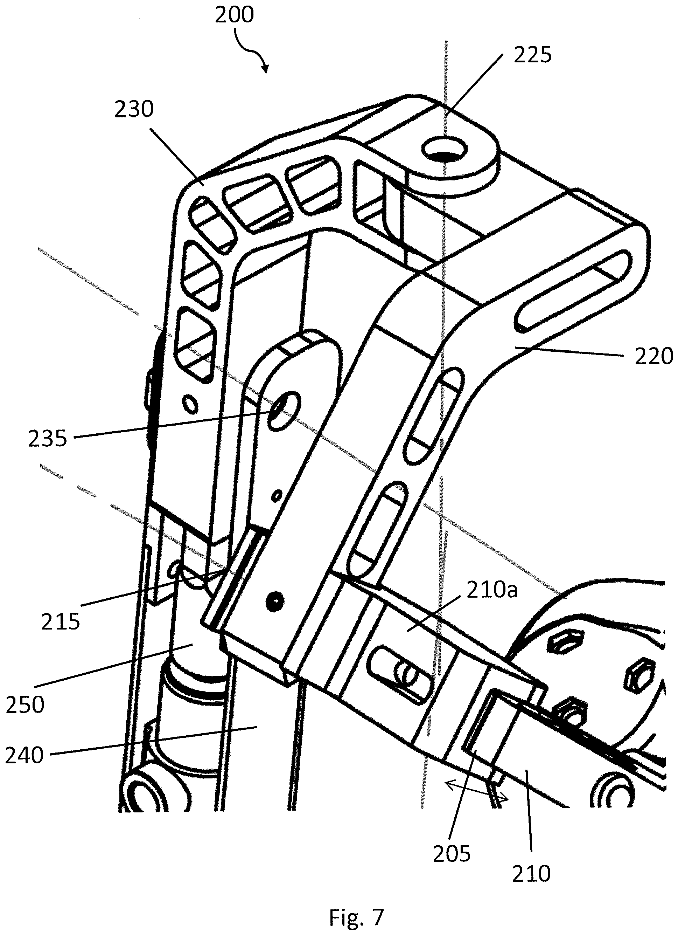

Fig. 5 und Fig. 7 zeigen eine Schultergelenksanordnung 200. Die Schultergelenksanordnung 200 weist folgende Komponenten auf, ein Schulteranlageelement 210, 210a, ein erstes Schulterkopplungselement 220, ein zweites Schulterkopplungselement 230 und eine Armstütze 240. Das Schulteranlageelement 210 kann zweiteilig ausgeführt sein, so dass es einen ersten Teil 210 und einen zweiten Teil 210a aufweist. Hierdurch kann eine translatorische Achse 205 bereitgestellt werden, da das eine Teil in das andere Teil hineingeschoben werden kann, um das Exoskelett an die Schulterbreite des Nutzers anpassen zu können. Die translatorisch Achse 205 kann in einer Ausführungsform nach dem Einstellen arretiert werden, so dass diese sich nicht während der Nutzung verstellt und die Kraft effektiv auf das Rückenteil und das Beckenstützelement übertragen werden kann. Das Schulteranlageelement 210 bzw. 210a ist mit dem ersten Schulterkopplungselement 220 rotatorisch verbunden, so dass eine erste Rotationsachse 215 entsteht. Die erste Rotationsachse 215 kann als Kippachse ausgeführt sein, welche näher in Fig. 6 beschrieben wird. Das erste Schulterkopplungselement 220 ist mit dem zweiten Schulterkopplungselement 230 über eine zweite Rotationsachse 225 verbunden. Das zweite Schulterkopplungselement 230 ist mit der Armstütze 240 über eine dritte Rotationsachse 235 verbunden. Die zweite Rotationsachse 225 und die dritte Rotationsachse 235 schneiden sich in einem Winkel zwischen 0° und 90°, insbesondere in einem Winkel von 85° ± 5°. Die zweite Rotationsachse 225 und die erste Rotationsachse 215 schneiden sich nicht direkt, da das erste Schulterkopplungselement 220 sowohl seitlich als auch nach hinten auskragt. Somit sind diese beiden Rotationsachsen zwar orthogonal zueinander aber beabstandet voneinander. Die oben beschriebene Schultergelenksanordnung 200 ermöglicht es mit einer Anzahl von drei Rotationsachsen 215, 225, 235 eine hohe Beweglichkeit im Schulterbereich des Nutzers sicherzustellen. Zwischen der Armstütze 240 und dem zweiten Schulterkopplungselement 230 kann ein Aktuator 250 angebracht sein, dieser kann ausgeführt sein, den Arm des Nutzers zu unterstützen. Somit kann der Nutzer längere Arbeiten mit Werkzeugen ausführen bzw. besser über Kopfhöhe arbeiten. Ferner kann für die Ansteuerung des Aktuators 250 ein Sensor die Kraft oder den Winkel messen, um eine ideale Unterstützung des Nutzers zu gewährleisten.

Fig. 6 zeigt eine Schnittansicht der ersten Rotationsachse 215. Ferner werden auf Fig. 6 das erste Schulterkopplungselement 220 und das Schulteranlageelement 210a dargestellt. Bei der ersten Rotationsachse 215 handelt es sich um eine Kippachse, welche in einem Winkel von 50° kippbar ist. Die Konstruktion sieht vor, dass mittels zweier Anschläge der gewünschte Winkel, hier 50°, eingestellt werden kann. Somit kann ein Überdehnen des Menschen verhindert werden.

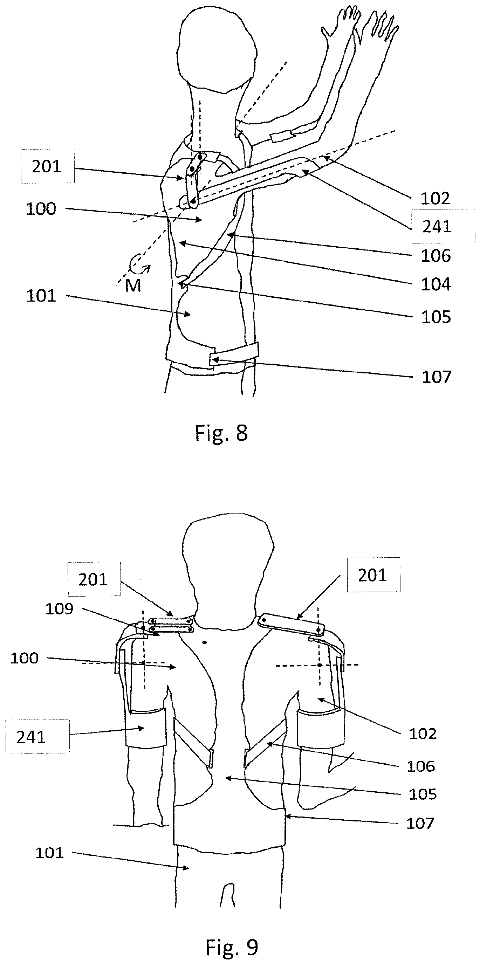

Figuren 8 bis 11 zeigen jeweils eine mögliche Ausprägungsform der Stützvorrichtung bzw. des Exoskeletts für den Anwendungskontext der Tätigkeiten in und über Kopfhöhe. Bei dieser Anwendung handelt es sich um einen ergonomisch sehr kritischen Anwendungsfall. Die Vorrichtung soll vor allem Mitarbeiter, beispielsweise in der Produktion, vor Überlastungsschäden, z.B. im Schulterbereich, verschonen (Use Case 1 Kraftumleitung), bzw. die Ausführung von Tätigkeiten in und über Kopfhöhe ermöglichen (Use Case 2 Kraftsteigerung).

Fig. 8 skizziert ein erstes Beispiel für eine Stützvorrichtung 100, deren Zusammenwirken mit dem Nutzer 101 und zwischen den Systemelementen dargestellt ist. Die Stützvorrichtung 100 ist über zwei Mensch-Technik-Schnittstellen mit dem Nutzer 101 gekoppelt: eine Schnittstelle zwischen der Stützvorrichtung 100 und dem Oberarm des Nutzers 102, z.B. eine Armstütze, bezeichnet als Schnittstelle zwischen technischem System und dem menschlichen Arm (Arm-System-Schnittstelle 241), sowie eine Schnittstelle zwischen der Stützvorrichtung 100 und dem menschlichen Torso 104, bezeichnet als Schnittstelle zwischen technischem System und dem menschlichen Torso (System-Torso-Schnittstelle 105), beispielsweise ein Beckenstützelement. Die System-Torso-Schnittstelle 105 ist mit einem Träger-/Verbindungssystem 106 ausgestattet. Darüber hinaus besitzt die System-Torso Schnittstelle 105 eine speziell auf den Nutzer 101 anzupassende Anschlussstelle zum menschlichen Becken 107. Darüber hinaus verfügt die Stützvorrichtung 100 über eine Vorrichtung zur Realisierung vor allem rotatorischer menschlicher Bewegungen 201, die zwischen der System-Torso-Schnittstelle 105 und der Arm-System-Schnittstelle 241 angeordnet ist. Diese mögliche Ausprägung der Vorrichtung zur Realisierung menschlicher Bewegungen 241 besitzt drei Freiheitsgrade. Eine Aktuierungseinheit bzw. ein Aktuator ist in Figur 8 nicht dargestellt, kann für den Einsatz jedoch erforderlich sein. Mögliche Ausprägungsformen für die Aktuierungseinheit und darüber hinaus für die notwendige Sensorik und Steuerungseinheit bei aktiven Aktuierungseinheiten ist oben dargestellt.

Fig. 9 zeigt eine zweite Skizze einer weiteren Ausprägungsform der erfindungsgemäßen Stützvorrichtung 100 mit vor allem einer anderen Ausprägung der Vorrichtung zur Realisierung rotatorischer und translatorischer menschlicher Bewegungen 201, wobei die Ausprägung aus Figur 8 rechts und die neue Variante links dargestellt ist. Die links dargestellte Ausprägungsform - teilweise Parallelkinematik - ermöglicht zusätzlich zu der Realisierung der rotatorischen menschlichen Bewegung zusätzlich die Realisierung einer translatorischen menschlichen Bewegung. Dieser spezielle Teil der Kinematik der Vorrichtung ist oberhalb der menschlichen Schulter 109 angeordnet.

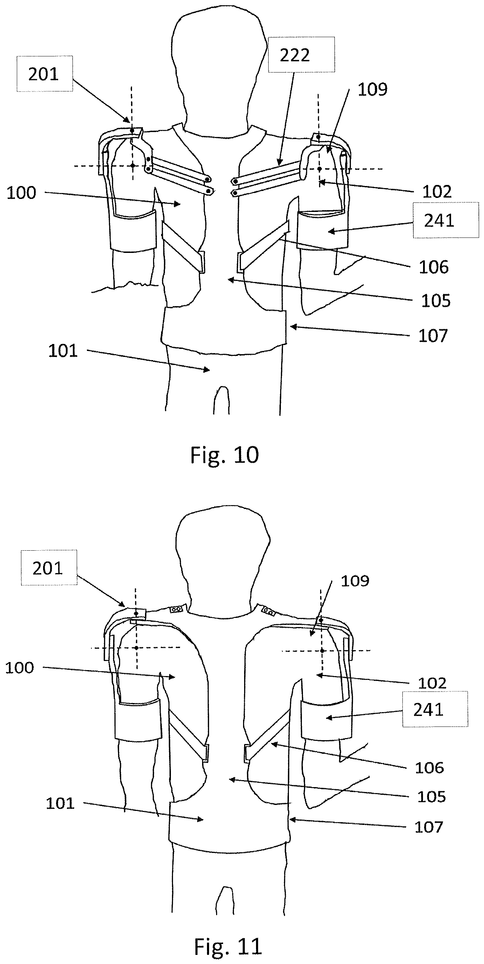

Eine weitere Ausprägungsform der Stützvorrichtung 100 ist in Fig. 10 dargestellt - Hauptunterschied ist hierbei in der Vorrichtung zur Realisierung rotatorischer und translatorischer menschlicher Bewegungen 201. Die Konstruktion dieser Vorrichtung ist im Vergleich zu den Ausführungsformen aus Figur 8 und Figur 9 grundsätzlich anders ausgeführt, um die Bewegungstreue zu verbessern. Eine am oberen Rücken des Nutzers angeordnete Parallelkinematik 222 bildet in der Vorrichtung zur Realisierung der menschlichen Bewegung die Funktion "anheben/hochziehen der Schulter" (zwei-dimensionale Verschiebung) ab. Die Rotation der Schulter wird durch ein zu den Ausprägungsformen aus Figur 8 und Figur 9 ähnliches Element realisiert (ein-dimensionale Rotation).

Eine vierte Ausführungsform für die Vorrichtung zur Realisierung menschlicher Bewegungen 201 ist in Figur 11 skizziert. Diese Ausführungsform besitzt zwei Freiheitsgrade, vor allem zur Realisierung rotatorischer menschlicher Bewegungen. Aufgrund der andersartigen Gestaltung der Vorrichtung zur Realisierung menschlicher Bewegungen 211 ist eine anders gestaltete System-Torso-Schnittstelle 105 erforderlich, speziell oberhalb der menschlichen Schulter 109.

Alle vier grob skizzierten Stützvorrichtungen bzw. Exoskelette 100 unterstützen zumindest einen Arm (speziell den oberen Arm 102) des Nutzers 101, der in den Skizzen direkt mit der Stützvorrichtung 100 über eine Schnittstelle gekoppelt ist. Unterschiedliche Varianten für die Vorrichtung zur Realisierung rotatorischer und translatorischer menschlicher Bewegungen 211 wurden skizziert. Varianten und Ausprägungsformen für die Integration und Ausführung der Aktuierungseinheit, und im Falle von aktiven Systemen, der Sensor- und Steuerungseinheit, wurden nicht dargestellt. Ausführungsformen wurden oben beschrieben.

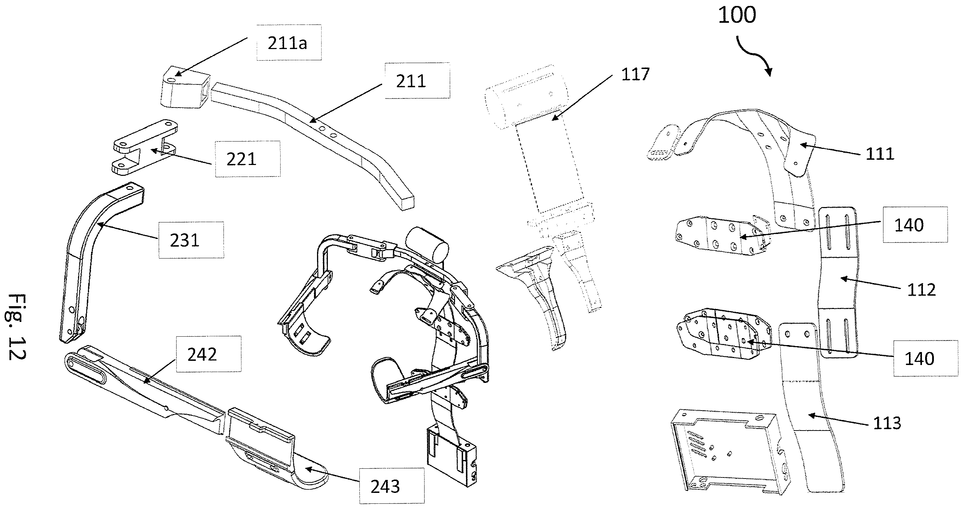

Eine darüber hinaus gehende Ausprägung ist in Figur 12 skizziert. Eine spezielle Ausprägung besitzt hier die Rückenkonstruktion bzw. das Rückenteil. Dieses besteht aus einem oberen Rückenelement 111, einem mittleren Rückenelement 112 und einem unteren Rückenelement 113. Das besondere hierbei ist, dass diese Konstruktion Einstellmöglichkeiten vorsieht, die speziell die Anpassung in der Höhe individuell ermöglicht. Das Profil der Rückenkonstruktion besitzt eine S-Form (in Analogie zur Form der Wirbelsäule). Die Rückenelemente können aus unterschiedlichen Materialien hergestellt werden, z.B. Kunststoff, CFK, GFK oder Metall. Es bietet sich hier allerdings speziell kohlefaserverstärkter Kunststoff (CFK) an, da hiermit besonders gut richtungsabhängige Steifigkeitseigenschaften eingestellt werden können. Somit ist es damit möglich eine so weiche Struktur zu bauen, die das Krümmen des Rückens ermöglicht, aber dennoch in der Lage ist die auftretenden Kräfte abzuleiten. Das obere Rückenelement 111 umschließend nur gerade so die Schultern. Ein weiteres umschließen ist nicht erforderlich, da hier keine Kräfte aufgenommen bzw. eingeleitet werden sollen, sondern vielmehr nur eine Referenzierung und Arretierung ermöglicht werden soll. Die Verbindung der Rückenelemente erfolgt z.B. per Schraubverbindungen.

Zusätzlich wird das Rückensystem mit einer Rucksackhalterung ausgestattet. Für die eine Anbindungsseite sind spezielle Elemente 140 vorgesehen. Ein weiteres Anbindungselement ist für einen Brust-Haltegurt erforderlich, der den Brustgurt des Nutzers umschließt - auch hier nur zur Referenzierung und Arretierung, nicht zur Krafteinleitung. Das untere Rückenelement 113 ist wiederum mit einem Beckengurt verbunden (nicht dargestellt). Am oberen Rückenelement 111 ist wiederum ein Anbindungselement 140 befestigt, welches die Anbindung zu den weiteren Systemteilen ermöglicht. Hierzu zählen zum einen die Armkinematik und zum anderen die Kopfstütze 117. Die grundlegende Verbindung zur Armkinematik stellt ein Umlenkungsbügel 118 dar, der mit dem Anbindungselement 140 verbunden ist. An diesem Umlenkbügel kann ein oder zwei Armelemente angebaut werden. Diese Armelemente bestehen aus vier mechanischen Elementen, einer Mensch-Technik-Anbindung und einem Aktuator (nicht abgebildet). Zu den vier Elementen gehört die Anbindung Arm-Umlenkbügel 211a, eine Gabelbrücke 221, ein 90° Umlenkbügel 231 und der Armhebel 242. Zwischen dem Armhebel 242 und dem 90° Umlenkbügel 231 wird der Aktuator verspannt. Als Aktuator kann z.B. eine Gasdruckfeder, ein Pneumatikaktuator oder ein Elektromotor genutzt werden. Zur Sollwertberechnung kann darüber hinaus auch noch Sensorik integriert werden. An den Armhebel 242 wird die Mensch-Technik-Schnittstelle 243 angebaut. Die Position kann über eine Stellvorrichtung eingestellt werden.

Für den Kontext, in dem sich die in Figuren 8 - 11 dargestellten Stützvorrichtungen anwenden lassen, ist es wichtig, dass dem Nutzer das Gewicht seiner eigenen Extremitäten und der gehaltenen Werkzeuge abgenommen wird. Insbesondere in statischen Situationen ist die menschliche Muskulatur auf Grund verminderter Durchblutung in der Leistungsfähigkeit gegenüber dynamischer Belastung beschränkt. Es ist hingegen in der Regel nicht notwendig den Nutzer bei der Positionierung zu unterstützen oder die Dynamik in horizontaler Richtung zu steigern. Daher wird bei dem hier beschriebenen Konzept durch die konstruktive Gestaltung gezielt in vertikaler Richtung unterstützt wohingegen in horizontaler Richtung möglichst wenig Kraft auf den Nutzer übertragen wird. Dies wird erreicht, indem passiv gestaltete Rotationsachsen parallel zur Gravitationsrichtung verlaufen. Aktiv oder passiv angetriebene Rotationsachsen verlaufen in einer zum Boden parallelen Ebene. Das an der Rotationachse anliegende Drehmoment wird dabei abhängig vom Winkel zwischen Körperlängsachse und der Längsachse der parallel zum unterstützten Körperteil verlaufenden mechanischen Struktur so gewählt, dass am Punkt des größten Hebelarms das größte Moment erreicht wird. Für ein am Oberarm angebrachtes Unterstützungssystem ergibt sich mit diesem Prinzip ein Antriebsmoment gemäß

$$M_{Antrieb} = \sin(phi_{Oberarm}) * M_{Antrieb,maximal}$$

aus einem festgelegten Maximalunterstützungsmoment $M_{Antrieb,maximal}$ mit einem Winkel $phi_{Oberarm}$ zwischen Körperlängsachse und Oberarm. Neben dem Winkel $phi_{Oberarm}$ können hierbei weitere physiologische Parameter wie der Verlauf der maximalen Muskelkraft über den Gelenkwinkel (Kraftkurvenverlauf des Muskels) in die Berechnung einfließen, um sicherzustellen, dass die Unterstützungskraft geringer ist als die für die Ausführung der Aufgabe notwendige Kraft. Das System bewegt den Nutzer somit nicht selbsttätig. Es ist stets notwendig, dass der Nutzer Kraft aufbringt. Für andere Körpergelenke können sinngemäß identische Zusammenhänge zwischen Gelenkwinkel und Unterstützungskraft aufgestellt werden. Durch geeignete Verknüpfung kann somit sichergestellt werden, dass dem Nutzer die schädliche Gewichtsbelastung zum Teil abgenommen wird, wohingegen die Bewegungsfreiheit erhalten bleibt.

Für vor allem für translatorische Bewegungen, wie sie beispielsweise am Schultergürtel auftreten, sind auch andere Bewegungsmuster denkbar. Hier ist aus physiologischen Gründen sinnvoll, die entgegen der Gravitation wirkende Kraft beim Überschreiten eines Winkels von 90° zwischen Oberarm und Körperlängsachse einzuschalten.

Weitere Sensorik (bspw. EMG-Sensoren) zur Integration einer Erkennung der Intention des Nutzers ist möglich. Das Konzept der vornehmlichen Kompensation von Gravitationseinflüssen wird dabei beibehalten und um eine situationsabhängige Beeinflussung der maximalen Unterstützungskraft $M_{Antrieb,maximal}$ erweitert.

Die beschriebene konstruktive Realisierung ermöglicht es die potentielle Energie vereinfacht zu speichern (bspw. als elastische Energie in Federn oder Druckluft) und bei Bedarf wieder an den Nutzer abzugeben.

Neben dieser Starrkörperbetrachtung ist es zudem wichtig festzustellen, dass die Stützvorrichtung aus flexiblen Elementen (insbes. im Bereich des Rückens) besteht. Diese sind so angebracht, dass sie in Bezug auf die Richtung der Haupt-Unterstützungskraft steif sind, in andere Richtung jedoch flexibel. Dadurch können sich diese an die Form des Körpers und die Bewegung (insbesondere Flexion des Rückens, vergl. System-Torso-Schnittstelle 105 in Figur 1) anpassen. Allgemein wird dies durch flache, nah am Körper verlaufende Strukturen erreicht. Durch Textilbefestigung (vergl. Träger-/Verbindungssystem 106 in Figur 9) um den betreffenden Körperteil herum, wird erreicht, dass sich die Struktur an das Körperteil anpasst und bei Belastung parallel zur Längsachse nicht wegknickt.

Bezugszeichenliste

| 100 | Exoskelett bzw. Stützvorrichtung |
| 101 | Nutzer |
| 102 | Oberarm des Nutzers |
| 104 | menschlicher Torso |
| 105 | System-Torso-Schnittstelle |
| 106 | Träger-/Verbindungssystem |
| 107 | menschliches Becken |
| 109 | menschliche Schulter |

| 110 | Rückenteil |
|---|---|
| 111 | erstes Rückenelement |
| 112 | zweites Rückenelement |
| 113 | drittes Rückenelement |
| 114 | Anbindungselement für Rucksackhalterung |
| 116 | Anbindungselement |
| 117 | Kopfstütze |
| 121 | erster Kraftangriffspunkt |
| 122 | zweiter Kraftangriffspunkt |
| 130 | Erste Versteifungseinrichtung |
| 131 | Zweite Versteifungseinrichtung |
| 135 | Erstes Versteifungselement (a, b) |
| 136 | Zweites Versteifungselement (a, b) |
| 140 | Verbindungselement |
| 150 | Beckenstützelement |
| 151 | Erster Drehpunkt des Beckenstützelements |
| 152 | Zweiter Drehpunkt des Beckenstützelements |
| 160 | Haltestruktur |
| 161 | Haltestruktur |
| 170 | Seilspannvorrichtung |
| 180 | Schulterauflage |
| 200 | Schultergelenksanordnung |
| 201 | Vorrichtung zur Realisierung menschlicher Bewegungen |
| 205 | Translatorische Achse |
| 210 | Schulteranlageelement |
| 211 | Umlenkungsbügel |
| 211a | Anbindung Arm-Umlenkbügel |
| 215 | Erste Rotationsachse |
| 220 | Erster Schulterkopplungselement |
| 221 | Gabelbrücke |
| 222 | Parallelkinematik einer Ausprägungsform der Vorrichtung zur Realisierung menschlicher Bewegungen |
| 225 | Zweite Rotationsachse |
| 230 | Zweites Schulterkopplungselement |
| 231 | 90° Umlenkbügel |
| 235 | Dritte Rotationsachse |
| 240 | Armstütze |
| 242 | Armhebel |
| 243 | Mensch-Technik-Schnittstelle |
| 250 | Armunterstützer (Aktuator) |

**Patentansprüche**

1. Exoskelett (100) für einen Menschen, aufweisend:

  - ein Schulteranlageelement (210);
  - wenigstens eine Armstütze (240);
  - ein Beckenstützelement (150);
  - ein Rückenteil (110), mit einem ersten Ende und einem zweiten Ende;
  wobei das Rückenteil (110) wenigstens ein erstes flächenflexibles Rückenelement (111) mit einem ersten und einem zweiten Ende und wenigstens eine erste Versteifungseinrichtung (130) mit einem ersten Versteifungselement (135) aufweist,
  wobei das erste flächenflexible Rückenelement (111) so ausgerichtet ist, dass dessen erstes Ende in Richtung des ersten Endes des Rückenteils (110) gerichtet ist und dessen zweites Ende in Richtung des zweiten Endes des Rückenteils (110) gerichtet ist,
  wobei das Rückenteil (110) mit dem ersten Ende an dem Schulteranlageelement (210) befestigt ist,
  wobei das Rückenteil (110) mit dem zweiten Ende an dem Beckenstützelement (150) befestigt ist,
  wobei die Armstütze (240) direkt oder indirekt an dem Schulteranlageelement (210) angelenkt ist,

wobei die erste Versteifungseinrichtung (130) ausgeführt ist, das erste flächenflexible Rückenelement (111) gezielt in einer Biegerichtung zu versteifen

wobei das Rückenteil (110) ferner ein zweites flächenflexibles Rückenelement (112) mit einem ersten Ende und einem zweiten Ende, sowie ein Verbindungselement (140a) aufweist,

wobei das zweite Ende des ersten flächenflexiblen Rückenelements (111) mit dem ersten Ende des zweiten flächenflexiblen Rückenelements (112) über das Verbindungselement (140a) verbunden ist,

wobei das zweite flächenflexible Rückenelement (112) eine zweite Versteifungseinrichtung (131) mit einem zweiten Versteifungselement (136) aufweist,

wobei die zweite Versteifungseinrichtung (131) ausgeführt ist, das zweite flächenflexible Rückenelement (112) gezielt in einer Biegerichtung zu versteifen.

wobei die erste Versteifungseinrichtung (130) und die zweite Versteifungseinrichtung (131) unterschiedlichen Steifigkeiten aufweisen.

2. Exoskelett (100) gemäß Anspruch 1,

wobei an dem Rückenteil (110) schulteranlagenelementseitig ein erster Kraftangriffspunkt (121) vorgesehen ist,
wobei an dem Rückenteil (110) beckenstützelementseitig ein zweiter Kraftangriffspunkt (122) vorgesehen ist,
wobei das erste Versteifungselement (135) ein Seil ist, das zwischen dem ersten Kraftangriffspunkt (121) und dem zweiten Kraftangriffspunkt (122) außerhalb der Biegelinie des ersten flächenflexiblen Rückenelements (111) derart gespannt ist, so dass die Biegekraft gezielt in eine Richtung vergrößert wird.

3. Exoskelett (100) gemäß einem der Ansprüche 1 und 2,
wobei wenigstens eines von dem ersten flächenflexiblen Rückenelement (111) und dem zweiten flächenflexiblen Rückenelement (112) in Bezug auf das Verbindungselement (140) längenverschiebbar angeordnet ist, so dass der Abstand zwischen dem ersten flächenflexiblen Rückenelement (111) und dem zweiten flächenflexiblen Rückenelement (112) veränderbar ist.

4. Exoskelett (100) gemäß einem der vorhergehenden Ansprüche,
wobei auf der dem Rückenteil (110) abgewandten Oberfläche des flächenflexiblen Rückenelements (111, 112) von der Oberfläche wenigstens eine auskragende Haltestruktur (160, 161) angeordnet ist, an welcher das Versteifungselement (135) der Versteifungseinrichtung (130) anliegt, so dass das Versteifungselement (135) beabstandet zu der Oberfläche des flächenflexiblen Rückenelements (111, 112) verläuft.

5. Exoskelett (100) gemäß einem der Ansprüche 2 bis 4,

wobei wenigstens die erste Versteifungseinrichtung (130) eine Seilspannvorrichtung (170) aufweist, die ausgeführt ist, die Seilspannung zu verändern,
wobei wenigstens die erste Versteifungseinrichtung (130, 131) insbesondere einen ersten Aktuator aufweist,
wobei die Seilspannvorrichtung (170) ausgeführt ist, mittels des ersten Aktuators die Seilspannung zu verändern,
wobei der erste Aktuator insbesondere ein Pneumatikzylinder, ein pneumatischer Muskel oder ein Elektromotor ist.

6. Exoskelett (100) gemäß Anspruch 5, ferner aufweisend:

- eine Sensoranordnung zur Messung insbesondere eines Winkels oder einer Kraft;
- eine Steuereinheit;

wobei die Steuereinheit ausgeführt ist, den ersten Aktuator der Seilspannvorrichtung (170) auf Basis von Sensordaten der Sensoranordnung zu steuern, so dass situationsbedingt die Seilspannung der Versteifungseinrichtung (130, 131) anpassbar ist.

7. Exoskelett (100) gemäß einem der vorhergehenden Ansprüche,
wobei das Rückenteil (110) und das Beckenstützelement (150) drehbar um eine erste Rotationsachse (151) verbunden sind, welche orthogonal auf der Oberfläche des Rückenteils steht.

8. Exoskelett (100) gemäß einem der vorhergehenden Ansprüche,
wobei das Rückenteil (110) und das Beckenstützelement (150) drehbar um eine zweite Rotationsachse (152) verbunden sind, welche quer zur Hauptrichtung des Rückenteils (110) in der Fläche des Rückenteils (110) verläuft.

**9.** Exoskelett (100) gemäß Anspruch 7 oder 8,

wobei das Exoskelett (100) ferner einen zweiten Aktuator aufweist,
wobei der zweite Aktuator ein erstes Ende und ein zweites Ende aufweist,
wobei das erste Ende des zweiten Aktuators mit dem Rückenteil (110) verbunden ist,
wobei das zweite Ende des zweiten Aktuators mit dem Beckenstützelement (150) verbunden ist,
wobei der zweite Aktuator ausgeführt ist, die Bewegung zwischen Rücken und Becken zu unterstützen.

**10.** Exoskelett (100) gemäß einem der vorhergehenden Ansprüche,
wobei wenigstens das erste flächenflexiblen Rückenelement (111) aus kohlefaserverstärktem Kunststoff bestehen.

**11.** Exoskelett (100) gemäß einem der vorhergehenden Ansprüche,

wobei das Exoskelett (100) eine Schultergelenksanordnung (200) aufweist,
wobei eine Schultergelenksanordnung (200) das Schulteranlageelement (210), ein erstes Schulterkopplungselement (220), ein zweites Schulterkopplungselement (230) und die Armstütze (240) aufweist,
wobei das erste Schulterkopplungselement (220) über eine erste Rotationsachse (215) mit dem Schulteranlageelement (210) verbunden ist,
wobei das erste Schulterkopplungselement (220) und das zweite Schulterkopplungselement (230) über eine zweite Rotationsachse (225) verbunden sind,
wobei das zweite Schulterkopplungselement (230) und die Armstütze (240) über eine dritte Rotationsachse (235) verbunden sind,
wobei die erste Rotationsachse (215) und die zweite Rotationsachse (225) rechtwinklig zueinander und beabstandet voneinander angeordnet sind,
wobei sich die zweite Rotationsachse (225) und die dritte Rotationsachse (235) schneiden,
wobei die Schultergelenksanordnung (200) insbesondere ferner eine translatorische Achse (205) entlang des Schulteranlageelements (210) aufweist,
wobei das erste Schulterkopplungselement (220) entlang dieser translatorischen Achse (205) verschiebbar ist,
wobei die erste Rotationsachse (215) insbesondere eine Kippachse ist, die in einem Winkeln zwischen 0° bis 50° kippbar ist, und/oder
wobei der Winkel zwischen der zweiten und dritten Rotationsachse insbesondere zwischen 0° und 90° ist.

**12.** Exoskelett (100) gemäß Anspruch 11, ferner aufweisend:

- einen dritten Aktuator;

wobei der dritte Aktuator ein erstes Ende und ein zweites Ende aufweist,
wobei das erste Ende des dritten Aktuators mit der Armstütze (240) verbunden ist,
wobei das zweite Ende des dritten Aktuators mit dem zweiten Schulterkopplungselement (230) verbunden ist,
wobei der dritte Aktuator ausgeführt ist, die Schulterbewegung zu unterstützen.

**13.** Exoskelett (100) gemäß einem der Ansprüche 7 bis 12,
wobei die rotatorische Kopplung eine Parallelogramm-Kopplungskomponente (222) oder eine Trapez-Kopplungskomponente aufweist.

**14.** Exoskelett (100) gemäß einem der vorhergehenden Ansprüche,
wobei wenigstens ein Referenzierungselement zum Festlegen einer definierten Position zwischen dem Exoskelett zum Exoskelettträger vorliegt, wobei wenigstens ein Arretierungselement zum Befestigen des Exoskeletts an zumindest einem Körperteil des Trägers des Exoskeletts vorliegt.

## Claims

**1.** Exoskeleton (100) for a human, comprising:

- a shoulder attachment element (210);
- at least one arm support (240);

- a pelvic support element (150);
- a back part (110) having a first end and a second end;

wherein the back part (110) has at least one first flexible-area back element (111), having a first and a second end, and at least one reinforcement means (130), having a first reinforcement element (135),

wherein the first flexible-area back element (111) is orientated in such a way that the first end thereof is directed towards the first end of the back part (110) and the second end thereof is directed towards the second end of the back part (110),

wherein the back part (110) is fastened by the first end to the shoulder attachment element (210),

wherein the back part (110) is fastened by the second end to the pelvic support element (150),

wherein the arm support (240) is articulated directly or indirectly to the shoulder attachment element (210),

wherein the first reinforcement means (130) is configured to reinforce the first flexible-area back element (111) in a bending direction in a targeted manner,

wherein the back part (110) further comprises a second flexible-area back element (112) having a first end and a second end, as well as a connecting element (140a),

wherein the second end of the first flexible-area back element (111) is connected to the first end of the second flexible-area back element (112) via the connecting element (140a),

wherein the second flexible-area back element (112) comprises a second reinforcement means (131) having a second reinforcement element (136),

wherein the second reinforcement means (131) is configured to reinforce the second flexible-area back element (112) in a bending direction in a targeted manner,

wherein the first reinforcement means (130) and the second reinforcement means (131) are of different rigidities.

2. Exoskeleton (100) according to claim 1,

wherein a first force application point (121) is provided on the back part (110) on the shoulder attachment element side,

wherein a second force application point (122) is provided on the back part (110) on the pelvic support element side,

wherein the first reinforcement element (135) is a cable, which is tensioned between the first force application point (121) and the second force application point (122) outside the flexure line of the first flexible-area back element (111) in such a way that the bending force is increased in one direction in a targeted manner.

3. Exoskeleton (100) according to either claim 1 or claim 2, wherein at least one of the first flexible-area back element (111) and the second flexible-area back element (112) is arranged longitudinally displaceably with respect to the connecting element (140), in such a way that the distance between the first flexible-area back element (111) and the second flexible-area back element (112) is adjustable.

4. Exoskeleton (110) according to any of the preceding claims,
wherein, on the surface of the flexible-area back element (111, 112) remote from the back part (110), at least one holding structure (160, 161) is arranged, which protrudes from the surface and against which the reinforcement element (135) of the reinforcement means (130) is positioned, in such a way that the reinforcement element (135) extends at a distance from the surface of the flexible-area back element (111, 112).

5. Exoskeleton (100) according to any of claims 2 to 4,

wherein at least the first reinforcement means (130) has a cable tensioning device (170) configured to adjust the cable tension,

wherein at least the first reinforcement means (130, 131) comprises in particular a first actuator,

wherein the cable tensioning device (170) is configured to adjust the cable tension by means of the first actuator,

wherein the first actuator is in particular a pneumatic cylinder, a pneumatic muscle or an electric motor.

6. Exoskeleton (100) according to claim 5, further comprising:

- a sensor arrangement for measuring in particular an angle or a force;
- a control unit;

wherein the control unit is configured to control the first actuator of the cable tensioning device (170) on the basis of sensor data of the sensor arrangement, in such a way that the cable tension of the reinforcement means (130, 131) can be adapted depending on the situation.

7. Exoskeleton (100) according to any of the preceding claims,
wherein the back part (110) and the pelvic support element (150) are connected rotatably about a first axis of rotation (151), which proceeds orthogonally from the surface of the back part.

8. Exoskeleton (100) according to any of the preceding claims,
wherein the back part (110) and the pelvic support element (150) are rotatably connected about a second axis of rotation (152), which extends within the area of the back part (110) transverse to the primary direction of the back part (110).

9. Exoskeleton (100) according to either claim 7 or claim 8, wherein the exoskeleton (100) further comprises a second actuator,

wherein the second actuator has a first end and a second end,
wherein the first end of the second actuator is connected to the back part (110),
wherein the second end of the second actuator is connected to the pelvic support element (150),
wherein the second actuator is configured to assist the movement between the back and the pelvis.

10. Exoskeleton (100) according to any of the preceding claims,
wherein at least the first flexible-area back element (111) consists of carbon-fibre-reinforced plastics material.

11. Exoskeleton (100) according to any of the preceding claims,

wherein the exoskeleton (100) comprises a shoulder joint arrangement (200),
wherein a shoulder joint arrangement (200) comprises the shoulder attachment element (210), a first shoulder coupling element (220), a second shoulder coupling element (230) and the arm support (240),
wherein the first shoulder coupling element (220) is connected to the shoulder attachment element (210) via a first rotation shaft (215),
wherein the first shoulder coupling element (220) and the second shoulder coupling element (230) are connected via a second rotation shaft (225),
wherein the second shoulder coupling element (230) and the arm support (240) are connected via a third rotation shaft (235),
wherein the first rotation shaft (215) and the second rotation shaft (225) are arranged mutually perpendicular and spaced apart,
wherein the second rotation shaft (225) and the third rotation shaft (235) intersect,
wherein the shoulder joint arrangement (200) in particular further comprises a translational shaft (205) along the shoulder attachment element (210),
wherein the first shoulder coupling element (220) is displaceable along this translational shaft (205),
wherein the first rotation shaft (215) is in particular a tilt axle tiltable at an angle between 0° and 50°, and/or
wherein the angle between the second and third rotation shaft is in particular between 0° and 90°.

12. Exoskeleton (100) according to claim 11, further comprising:

- a third actuator;
wherein the third actuator has a first end and a second end,
wherein the first end of the third actuator is connected to the arm support (240),
wherein the second end of the third actuator is connected to the second shoulder coupling element (230),
wherein the third actuator is configured to assist the shoulder movement.

13. Exoskeleton (100) according to any of claims 7 to 12,
wherein the rotary coupling comprises a parallelogram coupling component (222) or a trapezium coupling component.

14. Exoskeleton (100) according to any of the preceding claims,

wherein at least one referencing element is present for establishing a defined position between the exoskeleton and the exoskeleton wearer,
wherein at least one locking element is present for fixing the exoskeleton to at least one body part of the wearer of the exoskeleton.

**Revendications**

1. Exosquelette (100) destiné à un être humain, comportant :

   - un élément d'appui sur l'épaule (210) ;
   - au moins un élément de soutien du bras (240) ;
   - un élément de soutien du bassin (150) ;
   - une partie dorsale (110) avec une première extrémité et une seconde extrémité ;
   dans lequel la partie dorsale (110) comporte au moins un premier élément dorsal à surface souple (111) avec une première extrémité et une seconde extrémité et au moins un premier dispositif de raidissement (130) avec un premier élément de raidissement (135),
   dans lequel le premier élément dorsal à surface souple (111) est orienté de telle sorte que sa première extrémité est dirigée vers la première extrémité de la partie dorsale (110) et sa seconde extrémité est dirigée vers la seconde extrémité de la partie dorsale (110),
   dans lequel la première extrémité de la partie dorsale (110) est fixée à l'élément d'appui sur l'épaule (210),
   dans lequel la seconde extrémité de la partie dorsale (110) est fixée à l'élément de soutien du bassin (150),
   dans lequel l'élément de soutien du bras (240) est articulé directement ou indirectement sur l'élément d'appui sur l'épaule (210),
   dans lequel le premier dispositif de raidissement (130) est mis en oeuvre de façon à raidir le premier élément dorsal à surface souple (111) spécifiquement dans une direction de flexion,
   dans lequel la partie dorsale (110) comporte en outre un second élément dorsal à surface souple (112) avec une première extrémité et une seconde extrémité, ainsi qu'un élément de liaison (140a),
   dans lequel la seconde extrémité du premier élément dorsal à surface souple (111) est reliée à la première extrémité du second élément dorsal à surface souple (112) par l'intermédiaire de l'élément de liaison (140a),
   dans lequel le second élément dorsal à surface souple (112) comporte un second dispositif de raidissement (131) avec un second élément de raidissement (136),
   dans lequel le second dispositif de raidissement (131) est mis en oeuvre de façon à raidir le second élément dorsal à surface souple (112) spécifiquement dans une direction de flexion,
   dans lequel le premier dispositif de raidissement (130) et le second dispositif de raidissement (131) ont différents niveaux de raideur.

2. Exosquelette (100) selon la revendication 1,

   dans lequel un premier point d'application de force (121) est prévu sur la partie dorsale (110) côté élément d'appui sur l'épaule,
   dans lequel un second point d'application de force (122) est prévu sur la partie dorsale (110) côté élément de soutien du bassin,
   dans lequel le premier élément de raidissement (135) est un câble qui est tendu entre le premier point d'application de force (121) et le second point d'application de force (122) à l'extérieur de la ligne de flexion du premier élément dorsal à surface souple (111), de sorte que la force de flexion augmente spécifiquement dans une direction.

3. Exosquelette (100) selon l'une des revendications 1 et 2, dans lequel au moins un élément parmi le premier élément dorsal à surface souple (111) et le second élément dorsal à surface souple (112) est agencé de manière longitudinalement mobile par rapport à l'élément de liaison (140), de sorte que la distance entre le premier élément dorsal à surface souple (111) et le second élément dorsal à surface souple (112) peut être changée.

4. Exosquelette (100) selon l'une des revendications précédentes,
   dans lequel au moins une structure de retenue en saillie (160, 161) est agencée sur la surface de l'élément dorsal à surface souple (111, 112) opposée à la partie dorsale (110) de la surface, structure de retenue sur laquelle l'élément de raidissement (135) du dispositif de raidissement (130) est en appui, de sorte que l'élément de raidissement (135) s'étend à distance de la surface de l'élément dorsal à surface souple (111, 112).

5. Exosquelette (100) selon l'une des revendications 2 à 4,

dans lequel au moins le premier dispositif de raidissement (130) comporte un dispositif de tension de câble (170) qui est mis en oeuvre de façon à faire varier la tension de câble,

dans lequel au moins le premier dispositif de raidissement (130, 131) comporte en particulier un premier actionneur,

dans lequel le dispositif de tension de câble (170) est mis en oeuvre de façon à faire varier la tension de câble au moyen du premier actionneur,

dans lequel le premier actionneur est en particulier un vérin pneumatique, un muscle pneumatique ou un moteur électrique.

6. Exosquelette (100) selon la revendication 5, comportant en outre :

- un agencement de capteurs pour mesurer en particulier un angle ou une force ;
- une unité de commande ;

dans lequel l'unité de commande est mise en oeuvre de façon à commander le premier actionneur du dispositif de tension de câble (170) sur la base de données de capteurs de l'agencement de capteurs, de sorte que la tension de câble du dispositif de raidissement (130, 132) peut être adaptée en fonction de la situation.

7. Exosquelette (100) selon l'une des revendications précédentes,
dans lequel la partie dorsale (110) et l'élément de soutien du bassin (150) sont reliés de façon à pouvoir tourner autour d'un premier axe de rotation (151) qui est orthogonal à la surface de la partie dorsale.

8. Exosquelette (100) selon l'une des revendications précédentes,
dans lequel la partie dorsale (110) et l'élément de soutien du bassin (150) sont reliés de façon à pouvoir tourner autour d'un second axe de rotation (152) qui s'étend transversalement à la direction principale de la partie dorsale (110) dans la surface de la partie dorsale (110) .

9. Exosquelette (100) selon la revendication 7 ou 8,

dans lequel l'exosquelette (100) comporte en outre un deuxième actionneur,
dans lequel le deuxième actionneur comporte une première extrémité et une seconde extrémité,
dans lequel la première extrémité du deuxième actionneur est reliée à la partie dorsale (110),
dans lequel la seconde extrémité du deuxième actionneur est reliée à l'élément de soutien du bassin (150),
dans lequel le deuxième actionneur est mis en oeuvre de façon à soutenir le mouvement entre le dos et le bassin.

10. Exosquelette (100) selon l'une des revendications précédentes,
dans lequel au moins le premier élément dorsal à surface souple (111) est constitué d'une matière plastique renforcée par des fibres de carbone.

11. Exosquelette (100) selon l'une des revendications précédentes,

dans lequel l'exosquelette (100) comporte un agencement d'articulation d'épaule (200),
dans lequel l'agencement d'articulation d'épaule (200) comporte l'élément d'appui sur l'épaule (210), un premier élément de couplage d'épaule (220), un second élément de couplage d'épaule (230) et l'élément de soutien du bras (240),
dans lequel le premier élément de couplage d'épaule (220) est relié à l'élément d'appui sur l'épaule (210) par l'intermédiaire d'un premier axe de rotation (215),
dans lequel le premier élément de couplage d'épaule (220) et le second élément de couplage d'épaule (230) sont reliés par l'intermédiaire d'un deuxième axe de rotation (225),
dans lequel le second élément de couplage d'épaule (230) et l'élément de soutien du bras (240) sont reliés par l'intermédiaire d'un troisième axe de rotation (235),
dans lequel le premier axe de rotation (215) et le deuxième axe de rotation (225) sont agencés à angle droit l'un par rapport à l'autre et à distance l'un de l'autre,
dans lequel le deuxième axe de rotation (225) et le troisième axe de rotation (235) se coupent,
dans lequel l'agencement d'articulation d'épaule (200) comporte en particulier également un axe de translation (205) le long de l'élément d'appui sur l'épaule (210),
dans lequel le premier élément de couplage d'épaule (220) est mobile le long de cet axe de translation (205),
dans lequel le premier axe de rotation (215) est en particulier un axe de basculement qui peut basculer à un angle compris entre 0° et 50°, et/ou

dans lequel l'angle entre les deuxième et troisième axes de rotation est en particulier compris entre 0° et 90°.

12. Exosquelette (100) selon la revendication 11, comportant en outre :

- un troisième actionneur ;
dans lequel le troisième actionneur comporte une première extrémité et une seconde extrémité,
dans lequel la première extrémité du troisième actionneur est reliée à l'élément de soutien du bras (240),
dans lequel la seconde extrémité du troisième actionneur est reliée au second élément de couplage d'épaule (230),
dans lequel le troisième actionneur est mis en oeuvre de façon à soutenir le mouvement d'épaule.

13. Exosquelette (100) selon l'une des revendications 7 à 12, dans lequel le couplage en rotation comporte un composant de couplage en parallélogramme (222) ou un composant de couplage en trapèze.

14. Exosquelette (100) selon l'une des revendications précédentes,
dans lequel au moins un élément de référence est présent pour fixer une position définie entre l'exosquelette et le porteur de l'exosquelette, dans lequel au moins un élément d'arrêt est présent pour fixer l'exosquelette sur au moins une partie corporelle du porteur de l'exosquelette.

Fig. 1

Fig. 2

Fig. 3

Fig. 4

Fig. 5

∢ 50°

Fig. 6

Fig. 7

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014195373 A
- DE 102011076843
- JP 2012024557 B
- JP 20013176429 A